# EUROPÄISCHE PATENTSCHRIFT

(11) **EP 3 558 949 B1**
(45) Veröffentlichungstag und Bekanntmachung des Hinweises auf die Patenterteilung: **30.09.2020**
(21) Anmeldenummer: 17832927.2
(22) Anmeldetag: 12.12.2017
(51) Int. Cl.: C07D 239/06, C09D 183/12, C08G 77/08, C08G 77/46, C09J 183/04, C08L 83/04

(54) **AMIDIN-KATALYSATOR FÜR HÄRTBARE ZUSAMMENSETZUNGEN**
AMIDINE CATALYST FOR CURABLE COMPOSITIONS
CATALYSEUR DE TYPE AMIDINE POUR DES COMPOSITIONS DURCISSABLES

(30) Priorität: 21.12.2016 EP 16206023
(43) Veröffentlichungstag der Anmeldung: 30.10.2019
(73) Patentinhaber: Sika Technology AG, 6340 Baar (CH)
(72) Erfinder: CANNAS, Rita, 8600 Dübendorf (CH); BURCKHARDT, Urs, 8049 Zürich (CH)
(74) Vertreter: Sika Patent Attorneys
(86) Internationale Anmeldenummer: PCT/EP2017/082395
(87) Internationale Veröffentlichungsnummer: WO 2018/114476

(56) Entgegenhaltungen:
- EP-A1- 1 985 666
- WO-A1-2016/166336
- US-A- 3 251 854

## Beschreibung

### Technisches Gebiet

Die Erfindung betrifft Amidine und deren Verwendung als Katalysator für härtbare Zusammensetzungen, insbesondere auf der Basis von Silangruppen aufweisenden Polymeren.

### Stand der Technik

Härtbare Zusammensetzungen spielen eine bedeutende Rolle in vielen technischen Anwendungen, beispielsweise als Klebstoffe, Dichtstoffe oder Beschichtungen. Ihre Aushärtung wird durch Vernetzungsreaktionen bewirkt, welche über Reaktivgruppen wie beispielsweise Silangruppen, Isocyanatgruppen oder Epoxidgruppen, ablaufen, wobei diese nach einem Mischvorgang, durch Erwärmen oder durch Kontakt mit Feuchtigkeit mit sich selbst oder einem Reaktionspartner reagieren und so in der Zusammensetzung ein polymeres Netzwerk bilden. Zur Beschleunigung solcher Vernetzungsreaktionen werden häufig Katalysatoren eingesetzt. Sehr oft handelt es sich dabei um toxikologisch bedenkliche Stoffe, welche eine potentielle Gefährdung von Verarbeiter und Umwelt darstellen, insbesondere nach der Aushärtung der Zusammensetzung, wenn der Katalysator oder dessen Abbauprodukte durch Ausgasen, Migration oder Auswaschen freigesetzt werden.
Bei Raumtemperatur härtbare Zusammensetzungen auf Basis von Silangruppen aufweisenden Polymeren sind ausgeprägt mit dieser Problemstellung konfrontiert. Silangruppen aufweisende Polymere sind dabei insbesondere Polyorganosiloxane, welche gemeinhin als "Silicone" bzw. "Siliconkautschuke" bezeichnet werden, und Silangruppen aufweisende organische Polymere, welche auch als "silanfunktionelle Polymere", "silanmodifizierte Polymere" (SMP) oder "silanterminierte Polymere" (STP) bezeichnet werden. Ihre Vernetzung verläuft über die Kondensation von Silanolgruppen unter Ausbildung von Siloxanbindungen und wird klassischerweise mittels Organozinn-Verbindungen, wie insbesondere Dialkylzinn(IV)carboxylaten, katalysiert. Diese zeichnen sich durch eine sehr hohe Aktivität in Bezug auf die Silanol-Kondensation aus und sind sehr hydrolysebeständig; allerdings sind sie gesundheitsschädlich und stark wassergefährdend. Oft werden sie mit weiteren Katalysatoren kombiniert, hauptsächlich mit basischen Verbindungen wie insbesondere Aminen, welche vor allem die vorgeschaltete Hydrolyse der Silangruppen beschleunigen. Aufgrund einer stärkeren Gewichtung von EHS-Aspekten durch Berufsverbände und Verbraucher sowie schärferer staatlicher Regulierung werden seit einiger Zeit vermehrt Anstrengungen unternommen, die Organozinn-Verbindungen durch andere, weniger toxische Katalysatoren zu ersetzen. So wurden etwa Organotitanate, -zirkonate und -aluminate als alternative Metallkatalysatoren beschrieben. Diese haben aber zumeist eine geringere katalytische Aktivität in Bezug auf die Silanol-Kondensation und bewirken eine deutlich langsamere Vernetzung. Wegen ihrer mangelnden Hydrolysestabilität können sie beim Lagern der Zusammensetzung durch Restfeuchte der Inhaltsstoffe einen Grossteil ihrer Aktivität verlieren, wodurch sich die Aushärtung stark verlangsamt oder ganz zum Erliegen kommt.
Eine weitere bekannte Alternative zu Organozinn-Verbindungen stellen stark basische Stickstoff-Verbindungen aus der Klasse der Amidine und Guanidine dar, welche in Kombination mit den erwähnten Metall-Katalysatoren oder auch allein eingesetzt werden können. Viele der gebräuchlichen Amidin- und Guanidin-Katalysatoren, wie insbesondere 1,8-Diazabicyclo[5.4.0]undec-7-en (DBU) oder 1,1,3,3-Tetramethylguanidin (TMG), sind allerdings leichtflüchtige und geruchsintensive sowie ebenfalls gesundheitsschädliche und umweltgefährdende Stoffe. Ausserdem neigen sie dazu, aufgrund geringer Verträglichkeit mit der Zusammensetzung zu migrieren und dadurch Separation, Ausschwitzen oder Substratverschmutzung zu verursachen.
Weitere Amidin-Katalysatoren sind bekannt aus WO 2015/158859, EP 1 985 666 A1, WO 2015/158860, WO 2015/193208 und WO 2016/166336. Diese Katalysatoren sind aber bezüglich ihrer katalytischer Aktivität und/oder Verträglichkeit mit unterschiedlichen Polymer-Systemen noch verbesserungsfähig.

### Darstellung der Erfindung

Aufgabe der vorliegenden Erfindung ist es daher, einen Katalysator bereitzustellen, welcher die Nachteile des Standes der Technik überwindet und insbesondere geruchsarm und wenig toxisch ist, eine hohe katalytische Aktivität für die Vernetzungsreaktion von funktionellen Verbindungen besitzt und mit härtbaren Zusammensetzungen davon gut verträglich ist.
Diese Aufgabe wird durch ein Amidin enthaltend mindestens eine Struktureinheit der Formel (I) und Formel (II) wie in Anspruch 1 beschrieben gelöst. Das erfindungsgemässe Amidin ist weitgehend geruchlos und wenig toxisch. Es zeigt trotz erhöhtem Molekulargewicht eine überraschend hohe katalytische Aktivität für die Vernetzungsreaktion verschiedener funktioneller Verbindungen, insbesondere Verbindungen mit Silangruppen und/oder Isocyanatgruppen, und bewirkt eine rasche Aushärtung von härtbaren Zusammensetzungen davon. Das Amidin ist auf überraschend einfache Weise aus gut erhältlichen Grundstoffen herstellbar, wobei es über den Rest, an welchen die Struktureinheit der Formel (I) gebunden ist, hinsichtlich seiner Verträglichkeit mit unterschiedlichen härtbaren Zusammensetzungen optimal eingestellt werden kann, so dass es damit gut mischbar ist und sowohl vor als auch nach der Aushärtung kaum Separation oder Migration zeigt.
Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Amidin enthaltend mindestens eine Struktureinheit der Formel (I), 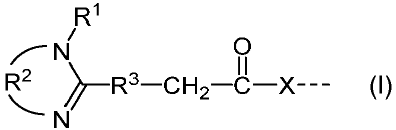 wobei
R¹ für einen Wasserstoff-Rest oder für einen gegebenenfalls ungesättigte Anteile aufweisenden Alkyl-, Cycloalkyl- oder Aralkyl-Rest mit 1 bis 30 C-Atomen, welcher gegebenenfalls eine tertiäre Aminogruppe aufweist, steht,
R² für einen gegebenenfalls Alkyl-substituierten 1,2-Ethylen- oder 1,3-Propylen-Rest steht,
R³ für einen gegebenenfalls Alkyl-substituierten 1,3-Propylen- oder 1,4-Butylen-Rest steht, und
X für O oder NR⁴ steht, wobei R⁴ für einen Wasserstoff-Rest oder für einen Alkyl-, Cycloalkyl- oder Aralkyl-Rest mit 1 bis 8 C-Atomen steht , dadurch gekennzeichnet, dass es die Formel (II) aufweist, wobei n für 1 oder 2 oder 3 steht, und A für einen n-wertigen Rest mit einem Molekulargewicht im Bereich von 28 bis 5'000 g/mol, welcher über C-Atome gebunden ist, steht. 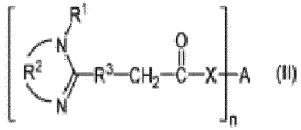
Eine gestrichelte Linie in den Formeln in diesem Dokument stellt jeweils die Bindung zwischen einem Substituenten und dem zugehörigen Molekülrest dar. Als "primäre Aminogruppe" bzw. "primärer Amin-Stickstoff" wird eine Aminogruppe bzw. deren Stickstoffatom bezeichnet, die bzw. das an einen einzigen organischen Rest gebunden ist und zwei Wasserstoffatome trägt; als "sekundäre Aminogruppe" bzw. "sekundärer Amin-Stickstoff" wird eine Aminogruppe bzw. deren Stickstoffatom bezeichnet, die bzw. das an zwei organische Reste, welche auch gemeinsam Teil eines Rings sein können, gebunden ist und ein Wasserstoffatom trägt; und als "tertiäre Aminogruppe" bzw. "tertiärer Amin-Stickstoff" wird eine Aminogruppe bzw. deren Stickstoffatom bezeichnet, die bzw. das an drei organische Reste, welche auch zu zweit oder zu dritt Teil eines oder mehrerer Ringe sein können, gebunden ist und kein Wasserstoffatom trägt.
Als "funktionelle Verbindung" wird eine Verbindung bezeichnet, welche mit einem geeigneten Reaktionspartner zu einer polymeren Struktur aushärtet und mindestens eine, insbesondere mindestens zwei, Reaktivgruppen trägt. Ihre Reaktivgruppen sind typischerweise elektrophil, während die Reaktionspartner nucleophil sind bzw. nucleophile Gruppen tragen.
Als "härtbar" wird eine Zusammensetzung bezeichnet, die durch Vernetzungsreaktionen von in ihr enthaltenen Reaktivgruppen aushärten bzw. einen Zustand erhöhter mechanischer Festigkeit erlangen kann.
Als "Siloxan-Rest" wird ein Rest enthaltend mindestens eine Siloxanbindung Si-O-Si bezeichnet.
Als "Polysiloxan-Rest" oder "Polyorganosiloxan-Rest" wird ein Siloxan-Rest enthaltend mehrere Siloxanbindungen in Folge, also Si-(O-Si)_{S}-Einheiten mit s = 2 oder mehr, bezeichnet. Dabei wird eine (O-Si)-Einheit als "Siloxan-Einheit" bezeichnet. Die Siloxan-Einheiten sind dabei zusätzlich mit organischen Resten, insbesondere Methyl- und/oder Phenylgruppen, substituiert.
Der Begriff "Silangruppe" bezeichnet eine an einen organischen Rest oder an einen Polysiloxan-Rest gebundene Silylgruppe mit einer bis drei, insbesondere zwei oder drei, hydrolysierbaren Substituenten am Siliciumatom. Besonders gebräuchliche hydrolysierbare Substituenten sind Alkoxy-Reste. Diese Silangruppen werden auch als "Alkoxysilangruppen" bezeichnet. Silangruppen können auch in partiell oder vollständig hydrolysierter Form vorhanden sein.
Als "Silan" werden sowohl Organoalkoxysilane, welche eine bis drei organische Substituenten an jeder Alkoxysilangruppe tragen, als auch Tetraalkoxysilane bezeichnet. Silane, die an einem organischen Rest zusätzlich zur Silangruppe eine oder mehrere Hydroxyl-, Isocyanato-, Amino- bzw. Mercaptogruppen tragen, werden als "Hydroxysilan", "Isocyanatosilan", "Aminosilan" bzw. "Mercaptosilan" bezeichnet.
Der Begriff "organisches Polymer" umfasst ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde und im PolymerRückgrat mehrheitlich Kohlenstoffatome aufweist, sowie Umsetzungsprodukte eines solchen Kollektivs von Makromolekülen. Polymere mit einem Polyorganosiloxan-Rückgrat (gemeinhin als "Silicone" bezeichnet) stellen keine organischen Polymere im Sinne des vorliegenden Dokuments dar.
Der Begriff "Silangruppen aufweisender Polyether" umfasst auch Silangruppen aufweisende organische Polymere, welche zusätzlich zu Polyether-Einheiten auch Urethangruppen, Harnstoffgruppen oder Thiourethangruppen enthalten können. Solche Silangruppen aufweisende Polyether können auch als "Silangruppen aufweisende Polyurethane" bezeichnet werden.
Als "Molekulargewicht" wird die molare Masse (in Gramm pro Mol) eines Moleküls bezeichnet. Als "mittleres Molekulargewicht" wird das Zahlenmittel Mₙ einer polydispersen Mischung von oligomeren oder polymeren Molekülen bezeichnet, welches üblicherweise mittels Gelpermeationschromatographie (GPC) gegen Polystyrol als Standard bestimmt wird.
Als "lagerstabil" oder "lagerfähig" wird eine Substanz oder eine Zusammensetzung bezeichnet, wenn sie bei Raumtemperatur in einem geeigneten Gebinde während längerer Zeit, typischerweise mindestens 3 Monaten bis zu 6 Monaten und mehr, aufbewahrt werden kann, ohne dass sie sich in ihren Anwendungs- oder Gebrauchseigenschaften, insbesondere der Viskosität und der Vernetzungsgeschwindigkeit, durch die Lagerung in einem für ihren Gebrauch relevanten Ausmass verändert.
Als "Raumtemperatur" wird eine Temperatur von ca. 23°C bezeichnet.

Die Struktureinheit der Formel (I) kann auch in protonierter Form vorliegen. Ebenfalls kann sie in komplexierter Form vorliegen, insbesondere mit Kationen von Zink, Eisen oder Molybdän.

Amidine enthaltend mindestens eine Struktureinheit der Formel (I), welche Siloxan-Einheiten aufweisen, sind besonders gut verträglich mit Polyorganosiloxan-Polymeren und somit besonders geeignet als Katalysator für solche funktionellen Verbindungen bzw. härtbare Zusammensetzungen.

Amidine enthaltend mindestens eine Struktureinheit der Formel (I), welche Polyoxyalkylen-Einheiten aufweisen, sind besonders gut verträglich mit Polyether-Polymeren und somit besonders geeignet als Katalysator für solche funktionellen Verbindungen bzw. härtbare Zusammensetzungen.

Amidine enthaltend mindestens eine Struktureinheit der Formel (I), welche eine tertiäre Aminogruppen aufweisen, sind besonders aktiv als Katalysator für Isocyanatgruppen und/oder Epoxidgruppen aufweisende Zusammensetzungen.

Bevorzugt steht R¹ für einen Wasserstoff-Rest oder für einen gegebenenfalls ungesättigte Anteile aufweisenden Alkyl- oder Cycloalkyl-Rest mit 1 bis 18 C-Atomen, welcher gegebenenfalls eine tertiäre Aminogruppe aufweist. Amidine mit solchen Struktureinheiten sind besonders einfach zugänglich.

Besonders bevorzugt ist R¹ ausgewählt aus der Gruppe bestehend aus Methyl, Ethyl, Propyl, Butyl, Cyclohexyl, 2-Ethylhexyl, n-Decyl, Lauryl, Cocoalkyl, Oleyl, Soyaalkyl, Talgalkyl und 3-(N,N-Dimethylamino)propyl. Amidine mit diesen Struktureinheiten sind ganz besonders einfach zugänglich.

Davon bevorzugt ist 3-(N,N-Dimethylamino)propyl. Amidine mit dieser Struktureinheit zeigen eine besonders hohe katalytische Aktivität.

Bevorzugt steht R² für einen gegebenenfalls Alkyl-substituierten 1,3-Propylen-Rest. Amidine mit einer solchen Struktureinheit zeigen eine besonders hohe katalytische Aktivität.
Am meisten bevorzugt steht R² für 1,3-Propylen.

Bevorzugt steht R³ für einen unsubstituierten 1,3-Propylen- oder 1,4-Butylen-Rest.
Am meisten bevorzugt steht R³ für 1,3-Propylen. Amidine mit solchen Struktureinheiten sind besonders einfach zugänglich und katalytisch besonders aktiv.

Bevorzugt steht X für NR⁴. Amidine mit solchen Struktureinheiten sind besonders stabil und können über den Rest, an welchen die Stuktureinheit gebunden ist, besonders gut variiert werden.

Bevorzugt steht R⁴ für einen Wasserstoff-Rest oder für einen Alkyl-Rest mit 1 bis 8, insbesondere 1 bis 4, C-Atomen.

Das Amidin enthaltend mindestens eine Struktureinheit der Formel (I) weist die Formel (II) auf, 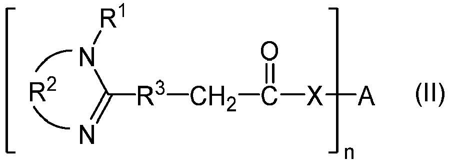 wobei
n für 1 oder 2 oder 3 steht,
A für einen n-wertigen Rest mit einem Molekulargewicht im Bereich von 28 bis 5'000 g/mol, welcher über C-Atome gebunden ist, steht,
und R¹, R², R³ und X die bereits genannten Bedeutungen aufweisen.

Bevorzugt steht A für einen einwertigen Kohlenwasserstoff-Rest mit 1 bis 30 C-Atomen, welcher gegebenenfalls Ether-Sauerstoff oder tertiären Amin-Stickstoff oder Siloxan-Einheiten enthält. Ein solches Amidin ist besonders niedrigviskos und einfach verarbeitbar.
Ein solcher einwertiger Rest A ist insbesondere ausgewählt aus der Gruppe bestehend aus Methyl, Ethyl, 1-Propyl, 2-Propyl, n-Butyl, 2-Butyl, Isobutyl, tert.Butyl, n-Pentyl, 2-Pentyl, Isopentyl, n-Hexyl, n-Octyl, 2-Ethylhexyl, n-Decyl, Lauryl, Cyclohexyl, Benzyl, 2-Methoxyethyl, 3-Methoxypropyl, 3-(N,N-Dimethylamino)propyl und Methylpolyoxyalkylen mit Oxyethylen- und/oder 1,2-Oxypropylen-Einheiten und einem mittleren Molekulargewicht im Bereich von 180 bis 600 g/mol.

Weiterhin bevorzugt steht A für einen zwei- oder dreiwertigen Kohlenwasserstoff-Rest mit 2 bis 50 C-Atomen, welcher gegebenenfalls Ether-Sauerstoff oder tertiären Amin-Stickstoff oder Siloxan-Einheiten enthält. Ein solches Amidin ist mit härtbaren Zusammensetzungen besonders gut verträglich und neigt besonders wenig zu Migration.
Bevorzugt steht X dabei für NR⁴ und R⁴ steht dabei bevorzugt für einen Wasserstoff-Rest oder für einen Methyl-Rest.
Ein solcher zwei- oder dreiwertiger Rest A ist insbesondere ausgewählt aus der Gruppe bestehend aus 1,2-Ethylen, 1,2-Propylen, 1,3-Propylen, 1,4-Butylen, 1,3-Pentlen, 1,5-Pentylen, 2,2-Dimethyl-1,3-propylen, 2-Methyl-1,5-pentylen, 1,6-Hexylen, 2,2(4),4-Trimethyl-1,6-hexamethylen, 1,8-Octylen, 1,10-Decylen, 1,12-Dodecylen, (1,5,5-Trimethylcyclohexan-1-yl)methan-1,3, 1,3-Cyclohexylen-bis(methylen), 1,4-Cyclohexylen-bis(methylen), 1,3-Phenylen-bis-(methylen), 2- und/oder 4-Methyl-1,3-cyclohexylen, N-Methyl-4-aza-1,7-heptylen, 3-Oxa-1,5-pentylen, 3,6-Dioxa-1,8-octylen, 4,7-Dioxa-1,10-decylen, α,ω-Polyoxypropylen mit einem mittleren Molekulargewicht im Bereich von 170 bis 2'000 g/mol, Trimethylolpropan- oder Glycerin-gestartetes Tris(ω)-polyoxypropylen) mit einem mittleren Molekulargewicht im Bereich von 330 bis 5'000 g/mol, Bis(3-propyl)-1,1,3,3-tetramethyldisiloxan und α,ω)-(1,3-Propylen)polydimethylsiloxan mit einem mittleren Molekulargewicht im Bereich von 350 bis 2'000 g/mol.

Davon bevorzugt ist 1,2-Ethylen, 1,2-Propylen, 1,3-Propylen, 1,4-Butylen,1,3-Pentylen, 1,5-Pentylen, 2,2-Dimethyl-1,3-propylen, 2-Methyl-1,5-pentylen, 1,6-Hexylen, 2,2(4),4-Trimethyl-1,6-hexamethylen, 1,8-Octylen, 1,10-Decylen, 1,12-Dodecylen, (1,5,5-Trimethylcyclohexan-1-yl)methan-1,3, 1,3-Cyclohexylen-bis(methylen), 1,4-Cyclohexylen-bis(methylen), 1,3-Phenylen-bis(methylen), 2- und/oder 4-Methyl-1,3-cyclohexylen oder N-Methyl-4-aza-1,7-heptylen. Diese Amidine der Formel (II) sind katalytisch besonders aktiv.
Davon weiterhin bevorzugt ist 3-Oxa-1,5-pentylen, 3,6-Dioxa-1,8-octylen, 4,7-Dioxa-1,10-decylen, α,ω-Polyoxypropylen mit einem mittleren Molekulargewicht im Bereich von 170 bis 2'000 g/mol oder Trimethylolpropan- oder Glycerin-gestartetes Tris(ω-polyoxypropylen) mit einem mittleren Molekulargewicht im Bereich von 330 bis 5'000 g/mol. Diese Amidine der Formel (II) sind besonders gut verträglich mit härtbaren Zusammensetzungen basierend auf Polyether-Polymeren, insbesondere Silangruppen aufweisenden Polyethern. Davon weiterhin bevorzugt ist Bis(3-propyl)-1,1,3,3-tetramethyldisiloxan oder α,ω-(1,3-Propylen)polydimethylsiloxan mit einem mittleren Molekulargewicht im Bereich von 350 bis 2'000 g/mol. Diese Amidine der Formel (II) sind besonders gut verträglich mit härtbaren Zusammensetzungen basierend auf Polyorganosiloxan-Polymeren.

Ein weiterer Gegenstand der Erfindung ist ein Verfahren zur Herstellung des Amidins der Formel (II), indem mindestens ein Amin **A1** der Formel (III) mit einer Verbindung der Formel (IV) unter Entfernung von Wasser umgesetzt wird, 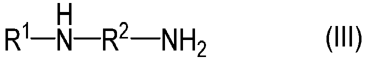 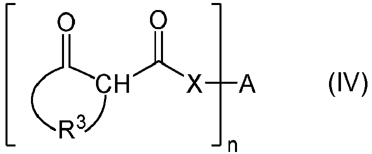 wobei R¹, R², R³, X, A und n die bereits genannten Bedeutungen aufweisen.

Das Verfahren ist überraschend schnell und einfach durchführbar, insbesondere ohne den Einsatz von Hilfsstoffen und ohne eine aufwendige Reinigung des Reaktionsprodukts zu erfordern, und geht von kommerziell zugänglichen, preisgünstigen Ausgangsmaterialien aus. Besonders überraschend ist dabei der Umstand, dass aus der cyclischen Verbindung der Formel (IV) unter Ringöffnung und erneutem Ringschluss unter milden Bedingungen auf einfache Weise ein cyclisches Amidin der Formel (II) entsteht.

Das Verfahren wird bevorzugt bei einer Temperatur im Bereich von 20 bis 120°C, besonders bevorzugt 30 bis 80°C, insbesondere 40 bis 60°C, durchgeführt, wobei die Reaktanden in beliebiger Reihenfolge miteinander vermischt werden können. Das Reaktionswasser wird nach der Umsetzung entfernt, bevorzugt durch Anlegen von Vakuum und/oder durch azeotrope Destillation mit einem organischen Lösemittel. Nach der Reaktion verbleibende flüchtige Bestandteile werden bevorzugt abdestilliert, insbesondere unter Vakuum. Ein solches Reaktionsprodukt kann ohne weitere Aufarbeitung oder Reinigung als Katalysator verwendet werden.

Der Einsatz einer Verbindung der Formel (IV), bei welcher X für NR⁴ steht, weist dabei den Vorteil auf, dass bei der Umsetzung mit dem Amin **A1** der Formel (III) weniger Nebenprodukte entstehen und das gebildete Reaktionsprodukt somit einen besonders hohen Gehalt an Amidin der Formel (II) aufweist.

Als Amin **A1** der Formel (III) geeignet sind insbesondere 1,2-Ethandiamin, N-Methyl-1,2-ethandiamin, N-Ethyl-1,2-ethandiamin, N-Propyl-1,2-ethandiamin, N-Isopropyl-1,2-ethandiamin, N-Butyl-1,2-ethandiamin, N-Cyclohexyl-1,2-ethandiamin, N-(2-Ethylhexyl)-1,2-ethandiamin, 1,2-Propandiamin, N-Methyl-1,2-propandiamin, N-Ethyl-1,2-propandiamin, N-Benzyl-1,2-propandiamin, 2-Methyl-1,2-propandiamin, 1,3-Propandiamin, N-Methyl-1,3-propandiamin, N-Ethyl-1,3-propandiamin, N-Propyl-1,3-propandiamin, N-Isopropyl-1,3-propandiamin, N-Butyl-1,3-propandiamin, N-Cyclohexyl-1,3-propandiamin, N-(2-Ethylhexyl)-1,3-propandiamin, N-Lauryl-1,3-propandiamin, N-Cocoalkyl-1,3-propandiamin, N-Oleyl-1,3-propandiamin, N-Soyaalkyl-1,3-propandiamin, N-Talgalkyl-1,3-propandiamin, 3-(3-(Dimethylamino)propylamino)propylamin, 2,2-Dimethyl-1,3-propandiamin, 1,3-Butandiamin, 1,3-Pentandiamin (DAMP), N-Methyl-1,3-pentandiamin, 1,2-Diaminocyclohexan, 1,3-Diaminocyclohexan oder 2(4)-Methyl-1,3-diaminocyclohexan.

Als Amin **A1** der Formel (III) bevorzugt sind 1,2-Ethandiamin, N-Methyl-1,2-ethandiamin, N-Ethyl-1,2-ethandiamin, N-Propyl-1,2-ethandiamin, N-Isopropyl-1,2-ethandiamin, N-Butyl-1,2-ethandiamin, N-Cyclohexyl-1,2-ethandiamin, 1,2-Propandiamin, 1,3-Propandiamin, N-Methyl-1,3-propandiamin, N-Ethyl-1,3-propandiamin, N-Propyl-1,3-propandiamin, N-Isopropyl-1,3-propandiamin, N-Butyl-1,3-propandiamin, N-Cyclohexyl-1,3-propandiamin, N-(2-Ethylhexyl)-1,3-propandiamin, N-Lauryl-1,3-propandiamin, N-Cocoalkyl-1,3-propandiamin, N-Oleyl-1,3-propandiamin, N-Soyaalkyl-1,3-propandiamin, N-Talgalkyl-1,3-propandiamin, 3-(3-(Dimethylamino)propylamino)propylamin, 2,2-Dimethyl-1,3-propandiamin, 1,3-Butandiamin oder 1,3-Pentandiamin (DAMP).

Als Amin **A1** der Formel (III) besonders bevorzugt sind 1,3-Propandiamin, N-Methyl-1,3-propandiamin, N-Ethyl-1,3-propandiamin, N-Butyl-1,3-propandiamin, N-Cyclohexyl-1,3-propandiamin, N-(2-Ethylhexyl)-1,3-propandiamin, N-Decyl-1,3-propandiamin, N-Lauryl-1,3-propandiamin oder 3-(3-(Dimethylamino)propylamino)propylamin.

Als Verbindung der Formel (IV) geeignet sind solche mit X = O. Solche cyclischen 1,3-Ketoester sind insbesondere Reaktionsprodukte aus der Dieckmann-Kondensation von Adipin- oder Pimelinsäure-diestern. Besonders geeignet sind 2-Oxocyclopentancarbonsäure-methylester, 2-Oxocyclopentancarbonsäure-ethylester, 2-Oxocyclohexancarbonsäure-methylester, 2-Oxocyclohexancarbonsäure-ethylester oder Analoge davon mit höheren Alkoholen, oder 1,2-Ethylen-bis(2-oxocyclopentanoat) oder Analoga von längerkettigen Diolen oder Polyalkylenoxid-Polyolen.

Als Verbindung der Formel (IV) bevorzugt sind solche mit X = NR⁴. Solche ein- oder mehrfunktionellen cyclischen 1,3-Ketoamide sind entweder direkt aus der Dieckmann-Kondensation von entsprechenden Adipin- oder Pimelinsäureamiden zugänglich, oder sie lassen sich durch Umsetzung der oben beschriebenen cyclischen 1,3-Ketoester mit mindestens einem Amin **A2** der Formel A-(NHR⁴)ₙ unter Freisetzung des entsprechenden Alkohols herstellen.

Bevorzugt wird für das beschriebene Verfahren eine Verbindung der Formel (IV) mit X = NR⁴ eingesetzt, welche vorgängig durch Umsetzung von mindestens einem Amin **A2** der Formel A-(NHR⁴)ₙ mit einer Verbindung der Formel (IV) mit X = O unter Entfernung des entsprechenden Alkohols erhalten wurde. Dabei weisen A, R⁴ und n die bereits genannten Bedeutungen auf.
Auf diesem Herstellungsweg kann die Struktur des Amidins der Formel (II) hinsichtlich dessen Verträglichkeit mit unterschiedlichen härtbaren Zusammensetzungen besonders einfach optimiert werden.

Die Verbindung der Formel (IV) mit X = O für diese vorgängige Umsetzung ist bevorzugt ausgewählt aus der Gruppe bestehend aus 2-Oxocyclopentancarbonsäure-methylester, 2-Oxocyclopentancarbonsäure-ethylester, 2-Oxocyclohexancarbonsäure-methylester und 2-Oxocyclohexancarbonsäure-ethylester.

Das Amin **A2** der Formel A-(NHR⁴)ₙ ist bevorzugt ausgewählt aus der Gruppe bestehend aus Dimethylamin, Diethylamin, Dipropylamin, N-Ethyl-N-propylamin, Diisopropylamin, N-Methyl-N-isopropylamin, 1-Butylamin, Dibutylamin, 2-Butylamin, Isobutylamin, tert.Butylamin, N-Methyl-N-butylamin, n-Pentylamin, 2-Pentylamin, Isopentylamin, n-Hexylamin, n-Octylamin, 2-Ethylhexylamin, Di-(2-ethylhexyl)amin, n-Decylamin, Laurylamin, Cocoalkylamin, Oleylamin, Soyaalkylamin, Talgalkylamin, Cyclohexylamin, Dicyclohexylamin, N-Methyl-N-cyclohexylamin, Benzylamin, N-Methyl-N-benzylamin, 2-Methoxyethylamin, 3-Methoxypropylamin, 3-(N,N-Dimethylamino)propylamin, Methylpolyoxyalkylenamin mit Oxyethylen- und/oder 1,2-Oxypropylen-Einheiten und einem mittleren Molekulargewicht im Bereich von 180 bis 600 g/mol, insbesondere Jeffamine® M-600 (von Huntsman), 1,2-Ethandiamin, 1,2-Propandiamin, 1,3-Propandiamin, 1,4-Butandiamin, 1,3-Pentandiamin, 1,5-Pentandiamin, 2,2-Dimethyl-1,3-propandiamin, 2-Methyl-1,5-pentandiamin, 1,6-Hexandiamin, 2,2(4),4-Trimethyl-1,6-hexandiamin, 1,8-Octandiamin, 1,10-Decandiamin, 1,12-Dodecandiamin, N-Methyl-1,2-ethandiamin, N-Ethyl-1,2-ethandiamin, N-Propyl-1,2-ethandiamin, N-Isopropyl-1,2-ethandiamin, N-Butyl-1,2-ethandiamin, N-Cyclohexyl-1,2-ethandiamin, N-Methyl-1,3-propandiamin, N-Ethyl-1,3-propandiamin, N-Propyl-1,3-propandiamin, N-Isopropyl-1,3-propandiamin, N-Butyl-1,3-propandiamin, N-Cyclohexyl-1,3-propandiamin, N-(2-Ethylhexyl)-1,3-propandiamin, N-Lauryl-1,3-propandiamin, N-Cocoalkyl-1,3-propandiamin, N-Oleyl-1,3-propandiamin, N-Soyaalkyl-1,3-propandiamin, N-Talgalkyl-1,3-propandiamin, 1-Amino-3-aminomethyl-3,5, 5-trimethylcyclohexan (Isophorondiamin oder IPDA), 1,3-Bis-(aminomethyl)cyclohexan, 1,4-Bis-(aminomethyl)cyclohexan, 1,3-Bis-(aminomethyl)benzol, 2- und/oder 4-Methyl-1,3-diaminocyclohexan, N,N-Bis(3-aminopropyl)methylamin, Bis-(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, α,ω-Polyoxypropylendiamine mit einem mittleren Molekulargewicht im Bereich von 200 bis 2'000 g/mol, insbesondere die Jeffamine® D-230, D-400, D-2000, SD-231, SD-401 und SD-2001 (von Huntsman), Trimethylolpropan- oder Glycerin-gestartetes Tris(ω-aminopolyoxypropylen) mit einem mittleren Molekulargewicht im Bereich von 330 bis 5'000 g/mol, insbesondere die Jeffamine® T-403, T-3000, T-5000 und ST-404 (von Huntsman), Bis(3-aminopropyl)-1,1,3,3-tetramethyldisiloxan und α,ω-(3-Aminopropyl)polydimethylsiloxan mit einem mittleren Molekulargewicht im Bereich von 350 bis 2'000 g/mol.

Ebenfalls bevorzugt ist ein Verfahren zur Herstellung eines Amidins der Formel (II), bei welchem n für 2 und X für NR⁴ steht, indem zwei mol Amidin der Formel (II) mit n = 1 und X = O mit einem mol Amin **A2,** welches zwei primäre oder sekundäre Aminogruppen aufweist, umgesetzt werden.

Als Verbindung der Formel (IV) besonders bevorzugt sind N-Butyl-(2-oxocyclopentyl)carbonamid, N-Butyl-(2-oxocyclohexyl)carbonamid, N-Hexyl-(2-oxocyclopentyl)carbonamid, N-Hexyl-(2-oxocyclohexyl)carbonamid, N,N-ethyl-(2-oxocyclopentyl)carbonamid, N,N-Diethyl-(2-oxocyclohexyl)carbonamid, N,N-Dibutyl-(2-oxocyclopentyl)carbonamid, N,N-Dibutyl-(2-oxocyclohexyl)carbonamid, N-Methyl-N-butyl-(2-oxocyclopentyl)carbonamid, N-Methyl-N-butyl-(2-oxocyclohexyl)carbonamid, N-Methyl-N-benzyl-(2-oxocyclopentyl)carbonamid, N-Methyl-N-benzyl-(2-oxocyclohexyl)carbonamid, N-(2-Ethylhexyl)-(2-oxocyclopentyl)carbonamid, N-(2-Ethylhexyl)-(2-oxocyclohexyl)carbonamid, N-Benzyl-(2-oxocyclopentyl)carbonamid, N-Benzyl-(2-oxocyclohexyl)carbonamid, N-Cyclohexyl-(2-oxocyclopentyl)carbonamid, N-Cyclohexyl-(2-oxocyclohexyl)carbonamid, N-(3-Dimethylaminopropyl)-(2-oxocyclopentyl)carbonamid, N-(3-Dimethylaminopropyl)-(2-oxocyclohexyl)carbonamid, N-(2-Methoxyethyl)-(2-oxocyclopentyl)carbonamid, N-(2-Methoxyethyl)-(2-oxocyclohexyl)carbonamid oder analoge Amide von Alkylpolyoxyalkylenaminen mit Oxyethylen- und/oder 1,2-Oxypropylen-Einheiten und einem mittleren Molekulargewicht im Bereich von etwa 180 bis 600 g/mol, oder Di- oder Triamide, wie insbesondere N,N'-Ethylen-bis((2-oxocyclopentyl)carbonamid), N,N'-Ethylen-bis((2-oxocyclohexyl)carbonamid) oder analoge Di- oder Triamide von höheren Di- oder Triaminen wie den vorgängig genannten Aminen der Formel A-(NHR⁴)ₙ mit n = 2 oder 3.

Ein weiterer Gegenstand der Erfindung ist die Verwendung des Amidins enthaltend mindestens eine Struktureinheit der Formel (I) und Formel (II) als Katalysator für die Vernetzung einer funktionellen Verbindung. Dabei katalysiert das Amidin die Vernetzungsreaktion der Reaktivgruppen bzw. die Aushärtung der funktionellen Verbindung und härtbaren Zusammensetzungen davon.

Bevorzugte Reaktivgruppen der funktionellen Verbindung sind Silangruppen, Isocyanatgruppen, Epoxidgruppen oder Cyanatestergruppen.

Als funktionelle Verbindung geeignet sind insbesondere
- Silane,
- Silangruppen aufweisende Polymere,
- Polyisocyanate,
- Isocyanatgruppen aufweisende Polymere, insbesondere Isocyanatgruppen aufweisende Polyurethanpolymere,
- Glycidoxygruppen aufweisende Verbindungen, insbesondere Epoxidharze,
- Cyanatesterharze, oder
- Polymere mit verschiedenen Reaktivgruppen, insbesondere Verbindungen oder Polymere mit Isocyanat- und Silangruppen oder mit Isocyanat- und Epoxidgruppen.

Die funktionelle Verbindung ist bevorzugt ein Silan, ein Silangruppen aufweisendes Polymer, ein Polyisocyanat oder ein Isocyanatgruppen aufweisendes Polyurethanpolymer.

Als Polyisocyanat geeignet sind insbesondere monomere Diisocyanate, Oligomere oder Polymere oder Derivate von monomeren Diiisocyanaten, oder beliebige Mischungen davon.

Geeignete monomere Diisocyanate sind insbesondere 2,4- oder 2,6-Toluylendiisocyanat oder beliebige Gemische dieser Isomeren (TDI), 4,4'-, 2,4'- oder 2,2'-Diphenylmethandiisocyanat oder beliebige Gemische dieser Isomeren (MDI), Gemische aus MDI und MDI-Homologen (polymeres MDI oder PMDI), 1,3- oder 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), 1,4-Tetramethylendiisocyanat, 2-Methylpentamethylen-1,5-diisocyanat, 1,6-Hexamethylendiisocyanat (HDI), 2,2(4),4-Trimethyl-1,6-hexamethylendiisocyanat (TMDI), 1,10-Decamethylendiisocyanat, 1,12-Dodecamethylendiisocyanat, Lysin-oder Lysinesterdiisocyanat, Cyclohexan-1,3- oder -1,4-diisocyanat, 1-Methyl-2,4- oder -2,6-diisocyanatocyclohexan oder beliebige Gemische dieser Isomeren (HTDI oder H₆TDI), 1-Isocyanato-3,3,5-trimethyl-5-isocyanatomethyl-cyclohexan (Isophorondiisocyanat oder IPDI), Perhydro-2,4'- oder -4,4'-diphenylmethandiisocyanat (HMDI oder H₁₂MDI), 1,4-Diisocyanato-2,2,6-trimethylcyclohexan (TMCDI), 1,3- oder 1,4-Bis-(isocyanatomethyl)cyclohexan, m- oder p-Xylylendiisocyanat (m- oder p-XDI), m- oder p-Tetramethyl-1,3- oder -1,4-xylylendiisocyanat (m- oder p-TMXDI) oder Bis-(1-Isocyanato-1-methylethyl)naphthalin.

Davon bevorzugt sind MDI, TDI, IPDI oder HDI.

Geeignete Oligomere, Polymere oder Derivate von monomeren Diisocyanaten sind insbesondere abgeleitet von MDI, TDI, HDI oder IPDI.

Als Polyisocyanat besonders bevorzugt sind bei Raumtemperatur flüssige Formen von MDI, welche einen hohen Gehalt an 4,4'-Diphenylmethandiisocyanat aufweisen. Sogenanntes "flüssiges MDI" stellt insbesondere entweder ein durch partielle chemische Modifizierung - insbesondere Carbodiimidisierung bzw. Uretoniminbildung - verflüssigtes 4,4'-Diphenylmethandiisocyanat dar, oder ist ein durch Abmischen gezielt herbeigeführtes oder durch den Herstellprozess bedingtes Gemisch von 4,4'-Diphenylmethandiisocyanat mit anderen MDI-Isomeren (2,4'-Diphenylmethandiisocyanat und/oder 2,2'-Diphenylmethandiisocyanat) oder MDI-Oligomeren oder MDI-Homologen.

Geeignete Isocyanatgruppen aufweisende Polyurethanpolymere werden insbesondere erhalten aus der Umsetzung von mindestens einem Polyol mit einer überstöchiometrischen Menge von mindestens einem Polyisocyanat, insbesondere einem Diisocyanat. Die Umsetzung wird bevorzugt unter Feuchtigkeitsausschluss bei einer Temperatur im Bereich von 50 bis 160°C durchgeführt, gegebenenfalls in Anwesenheit geeigneter Katalysatoren. Der Überschuss an Polyisocyanat wird bevorzugt so gewählt, dass im Polyurethanpolymer nach der Umsetzung aller Hydroxylgruppen ein Gehalt an freien Isocyanatgruppen im Bereich von 1 bis 30 Gewichts-%, bevorzugt 1.5 bis 25 Gewichts-%, besonders bevorzugt 2 bis 20 Gewichts-%, verbleibt. Gegebenenfalls kann das Polyurethanpolymer unter Mitverwendung von Weichmachern oder Lösemitteln hergestellt werden, wobei die verwendeten Weichmacher oder Lösemittel keine gegenüber Isocyanaten reaktive Gruppen enthalten.

Dafür geeignete Diisocyanate sind insbesondere MDI, TDI, PMDI, HDI, IPDI, H₁₂MDI, oder Oligomere oder Derivate dieser Diisocyanate.

Dafür geeignete Polyole sind insbesondere Polyetherpolyole, bevorzugt Polyoxyalkylenpolyole, welche Polymerisationsprodukte von Ethylenoxid oder 1,2-Propylenoxid oder 1,2- oder 2,3-Butylenoxid oder Oxetan oder Tetrahydrofuran oder Mischungen davon sind, eventuell polymerisiert mit Hilfe eines Startermoleküls mit zwei oder mehreren aktiven Wasserstoffatomen; Polyesterpolyole, bevorzugt Produkte aus der Polykondensation von Diolen oder Triolen mit Lactonen oder Dicarbonsäuren oder deren Estern oder Anydriden; Polycarbonatpolyole; OH-endständige Blockcopolymere mit mindestens zwei unterschiedlichen Blöcken mit Polyether-, Polyester- oder Polycarbonat-Einheiten; Polyacrylat- oder Polymethacrylat-Polyole; Polyhydroxy-funktionelle Fette oder Öle, insbesondere natürliche Fette oder Öle; oder Polykohlenwasserstoffpolyole wie beispielsweise Polyhydroxy-funktionelle Polyolefine, insbesondere Polybutadienpolyole.
Geeignet sind insbesondere auch Mischungen der genannten Polyole. Geeignet sind insbesondere Diole oder Triole oder Mischungen davon.

Das Isocyanatgruppen aufweisende Polyurethanpolymer weist bevorzugt ein mittleres Molekulargewicht im Bereich von 350 bis 30'000 g/mol, insbesondere 1'000 bis 15'000 g/mol, auf.

Als funktionelle Verbindung besonders bevorzugt sind Silane und/oder Silangruppen aufweisende Polymere.
Das Amidin enthaltend mindestens eine Struktureinheit der Formel (I) wirkt stark katalytisch auf die Hydrolyse- und Kondensationsreaktionen von Silangruppen. Silane und Silangruppen aufweisende Polymere härten deshalb schon mit einer relativ geringen Menge dieses Katalysators rasch aus.

Als Silan geeignet sind insbesondere Tetramethoxysilan, Tetraethoxysilan, Methyltrimethoxysilan, Ethyltrimethoxysilan, Propytrimethoxysilan, Octyltrimethoxysilan, Isooctyltrimethoxysilan, Vinyltrimethoxysilan, Phenyltrimethoxysilan, Methyltriethoxysilan, Octyltriethoxysilan, Isooctyltriethoxysilan, Vinyltriethoxysilan, Phenyltriethoxysilan, Aminosilane wie insbesondere 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropyldimethoxymethylsilan, N-(2-Aminoethyl)-N'-[3-(trimethoxysilyl)propyl]ethylendiamin oder deren Analoga mit Ethoxy- anstelle von Methoxygruppen, oder N-Phenyl-, N-Cyclohexyl- oder N-Alkylaminosilane, Mercaptosilane, Epoxysilane wie insbesondere 3-Glycidoxypropyltrimethoxysilan oder 3-Glycidoxypropyltriethoxysilan, oder 3-Ureidopropyltrimethoxysilan, (Meth)acrylosilane, Anhydridosilane, Carbamatosilane, oder Iminosilane, Addukte aus primären Aminosilanen mit Epoxysilanen oder (Meth)acrylosilanen oder Anhydridosilanen, oder oligomere Formen dieser Silane.

Als Silangruppen aufweisendes Polymer geeignet ist insbesondere ein Polyorganosiloxan mit endständigen Silangruppen oder ein Silangruppen aufweisendes organisches Polymer.

Ein bevorzugtes Polyorganosiloxan mit endständigen Silangruppen weist die Formel (V) auf, 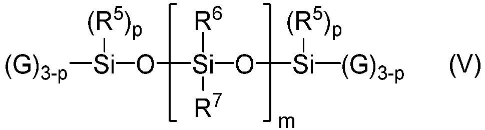 wobei
R⁵, R⁶ und R⁷ unabhängig voneinander jeweils für einen einwertigen Kohlenwasserstoff-Rest mit 1 bis 12 C-Atomen stehen,
G für einen Hydroxyl-Rest oder für einen Alkoxy-, Acetoxy-, Ketoximato-, Amido- oder Enoxy-Rest mit 1 bis 13 C-Atomen steht,
p für 0, 1 oder 2 steht, und
m für eine ganze Zahl im Bereich von 50 bis etwa 2'500 steht.

R⁵ steht bevorzugt für Methyl, Vinyl oder Phenyl.
R⁶ und R⁷ stehen bevorzugt unabhängig voneinander jeweils für einen Alkyl-rest mit 1 bis 5, bevorzugt 1 bis 3 C-Atomen, insbesondere für Methyl.
G steht bevorzugt für einen Hydroxyl-Rest oder für einen Alkoxy- oder Ketoximato-Rest mit 1 bis 6 C-Atomen, insbesondere für einen Hydroxyl-, Methoxy-, Ethoxy-, Methylethylketoximato- oder Methylisobutylketoximato-Rest. Besonders bevorzugt steht G für einen Hydroxylrest.
p steht bevorzugt für 0 oder 1, insbesondere für 0.
Weiterhin ist m bevorzugt so gewählt, dass das Polyorganosiloxan der Formel (V) bei Raumtemperatur eine Viskosität im Bereich von 100 bis 500'000 mPa·s, insbesondere von 1'000 bis 100'000 mPa·s, aufweist.

Polyorganosiloxane der Formel (V) sind gut handhabbar und vernetzen mit Feuchtigkeit und/oder Silanvernetzern zu festen Silicon-Polymeren mit elastischen Eigenschaften.
Geeignete kommerziell erhältliche Polyorganosiloxane der Formel (V) sind beispielsweise erhältlich von Wacker, Momentive Performance Materials, GE Advanced Materials, Dow Corning, Bluestar Silicones oder Shin-Etsu.

Als Silangruppen aufweisendes organisches Polymer geeignet ist insbesondere ein Polyolefin, Polyether, Polyester, Polyamide, Poly(meth)acrylat oder Mischformen dieser Polymere, welche jeweils eine oder bevorzugt mehrere Silangruppen tragen. Die Silangruppen können seitlich in der Kette oder endständig stehen und sind über ein C-Atom an das organische Polymer gebunden.

Besonders bevorzugt ist das Silangruppen aufweisende organische Polymer ein Silangruppen aufweisendes Polyolefin oder ein Silangruppen aufweisender Polyether oder ein Silangruppen aufweisender Polyester oder ein Silangruppen aufweisendes Poly(meth)acrylat oder eine Mischform dieser Polymere.

Am meisten bevorzugt ist das Silangruppen aufweisende organische Polymer ein Silangruppen aufweisender Polyether.

Das Silangruppen aufweisende organische Polymer weist als Silangruppen bevorzugt Alkoxysilangruppen auf, insbesondere Alkoxysilangruppen der Formel (VI), 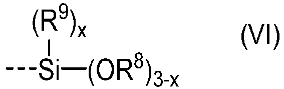 wobei
R⁸ für einen linearen oder verzweigten, einwertigen Kohlenwasserstoffrest mit 1 bis 5 C-Atomen, insbesondere für Methyl, Ethyl oder Isopropyl, steht,
R⁹ für einen linearen oder verzweigten, einwertigen Kohlenwasserstoffrest mit 1 bis 8 C-Atomen, insbesondere für Methyl, steht, und
x für einen Wert von 0 oder 1 oder 2, bevorzugt für 0 oder 1, insbesondere für 0, steht.

Besonders bevorzugt steht R⁸ für Methyl oder Ethyl.
Besonders bevorzugt sind Trimethoxysilangruppen, Dimethoxymethylsilangruppen oder Triethoxysilangruppen.
Dabei weisen Methoxysilangruppen den Vorteil auf, dass sie besonders reaktiv sind und schnell vernetzen, und Ethoxysilangruppen weisen den Vorteil auf, dass sie besonders lagerstabil sind und bei der Vernetzung wenig toxisches Ethanol freisetzen.

Das Silangruppen aufweisende organische Polymer weist im Mittel bevorzugt 1.3 bis 4, insbesondere 1.5 bis 3, besonders bevorzugt 1.7 bis 2.8, Silangruppen pro Molekül auf. Die Silangruppen sind bevorzugt endständig.

Das Silangruppen aufweisende organische Polymer weist bevorzugt ein mittleres Molekulargewicht im Bereich von 1'000 bis 30'000 g/mol, insbesondere 2'000 bis 20'000 g/mol, auf.

Das Silangruppen aufweisende organische Polymer weist bevorzugt ein Silan-Equivalentgewicht von 300 bis 25'000 g/Eq, insbesondere 500 bis 15'000 g/Eq, auf.

Das Silangruppen aufweisende organische Polymer kann bei Raumtemperatur fest oder flüssig vorliegen. Bevorzugt ist es bei Raumtemperatur flüssig.

Meist bevorzugt handelt es sich beim Silangruppen aufweisenden organischen Polymer um einen bei Raumtemperatur flüssigen Silangruppen aufweisenden Polyether, wobei die Silangruppen insbesondere Dialkoxysilangruppen und/oder Trialkoxysilangruppen, besonders bevorzugt Trimethoxysilangruppen oder Triethoxysilangruppen, sind.

Verfahren zur Herstellung von Silangruppen aufweisenden Polyethern sind dem Fachmann bekannt.

In einem bevorzugten Verfahren sind Silangruppen aufweisende Polyether erhältlich aus der Umsetzung von Allylgruppen-haltigen Polyethern mit Hydrosilanen, gegebenenfalls unter Kettenverlängerung mit beispielsweise Diisocyanaten.
In einem weiteren bevorzugten Verfahren sind Silangruppen aufweisende Polyether erhältlich aus der Copolymerisation von Alkylenoxiden und Epoxysilanen, gegebenenfalls unter Kettenverlängerung mit beispielsweise Diisocyanaten.
In einem weiteren bevorzugten Verfahren sind Silangruppen aufweisende Polyether erhältlich aus der Umsetzung von Polyetherpolyolen mit Isocyanatosilanen, gegebenenfalls unter Kettenverlängerung mit Diisocyanaten.
In einem weiteren bevorzugten Verfahren sind Silangruppen aufweisende Polyether erhältlich aus der Umsetzung von Isocyanatgruppen aufweisenden Polyethern, insbesondere NCO-terminierten Urethan-Polyethern aus der Umsetzung von Polyetherpolyolen mit einer überstöchiometischen Menge an Polyisocyanaten, mit Aminosilanen, Hydroxysilanen oder Mercaptosilanen. Silangruppen aufweisende Polyether aus diesem Verfahren sind besonders bevorzugt. Dieses Verfahren ermöglicht den Einsatz einer Vielzahl von kommerziell gut verfügbaren, kostengünstigen Ausgangsmaterialien, womit unterschiedliche Polymereigenschaften erhalten werden können, beispielsweise eine hohe Dehnbarkeit, eine hohe Festigkeit, ein niedriges Elastizitätsmodul, ein tiefer Glasübergangspunkt oder eine hohe Witterungsbeständigkeit.

Besonders bevorzugt ist der Silangruppen aufweisende Polyether erhältlich aus der Umsetzung von NCO-terminierten Urethan-Polyethern mit Aminosilanen oder Hydroxysilanen. Geeignete NCO-terminierte Urethan-Polyether sind erhältlich aus der Umsetzung von Polyetherpolyolen, insbesondere Polyoxyalkylendiolen oder Polyoxyalkylentriolen, bevorzugt Polyoxypropylendiolen oder Polyoxypropylentriolen, mit einer überstöchiometrischen Menge an Polyisocyanaten, insbesondere Diisocyanaten.
Bevorzugt wird die Umsetzung zwischen dem Polyisocyanat und dem Polyetherpolyol unter Feuchtigkeitsausschluss bei einer Temperatur von 50°C bis 160°C durchgeführt, gegebenenfalls in Anwesenheit geeigneter Katalysatoren, wobei das Polyisocyanat so dosiert ist, dass dessen Isocyanatgruppen im Verhältnis zu den Hydroxylgruppen des Polyols im stöchiometrischen Überschuss vorhanden sind. Insbesondere wird der Überschuss an Polyisocyanat so gewählt, dass im resultierenden Urethan-Polyether nach der Umsetzung aller Hydroxylgruppen ein Gehalt an freien Isocyanatgruppen von 0.1 bis 5 Gewichts-%, bevorzugt 0.2 bis 4 Gewichts-%, besonders bevorzugt 0.3 bis 3 Gewichts-%, verbleibt.
Bevorzugte Diisocyanate sind ausgewählt aus der Gruppe bestehend aus HDI, IPDI, TDI und MDI. Besonders bevorzugt sind IPDI oder TDI. Meist bevorzugt ist IPDI. Damit werden Silangruppen aufweisende Polyether mit besonders guter Lichtechtheit erhalten.

Speziell geeignet als Polyetherpolyole sind Polyoxyalkylendiole oder Polyoxyalkylentriole mit einem Ungesättigtheitsgrad tiefer als 0.02 mEq/g, insbesondere tiefer als 0.01 mEq/g, und einem mittleren Molekulargewicht im Bereich von 400 bis 25'000 g/mol, insbesondere 1'000 bis 20'000 g/mol.
Neben Polyetherpolyolen können anteilig auch andere Polyole eingesetzt werden, insbesondere Polyacrylatpolyole, sowie niedrigmolekulare Diole oder Triole.

Geeignete Aminosilane für die Umsetzung mit einem NCO-terminierten Urethan-Polyether sind primäre und sekundäre Aminosilane. Bevorzugt sind 3-Aminopropyltrimethoxysilan, 3-Aminopropyldimethoxymethylsilan, 4-Aminobutyltrimethoxysilan, 4-Amino-3-methylbutyltrimethoxysilan, 4-Amino-3,3-dimethylbutyltrimethoxysilan, N-Butyl-3-aminopropyltrimethoxysilan, N-Phenyl-3-aminopropyltrimethoxysilan, Addukte aus primären Aminosilanen wie 3-Aminopropyltrimethoxysilan, 3-Aminopropyldimethoxymethylsilan oder N-(2-Aminoethyl)-3-aminopropyltrimethoxysilan und Michael-Akzeptoren wie Acrylnitril, (Meth)acrylsäureestern, (Meth)acrylsäureamiden, Maleinsäure- oder Fumarsäurediestern, Citraconsäurediestern oder Itaconsäurediestern, insbesondere N-(3-Trimethoxysilylpropyl)aminobernsteinsäuredimethyl- oder -diethylester. Ebenfalls geeignet sind Analoga der genannten Aminosilane mit Ethoxy- oder Isopropoxygruppen anstelle der Methoxygruppen am Silicium.

Geeignete Hydroxysilane für die Umsetzung mit einem NCO-terminierten Urethan-Polyether sind insbesondere erhältlich aus der Addition von Aminosilanen an Lactone oder an cyclische Carbonate oder an Lactide.
Dafür geeignete Aminosilane sind insbesondere 3-Aminopropyltrimethoxysilan, 3-Aminopropyltriethoxysilan, 4-Aminobutyltrimethoxysilan, 4-Aminobutyltriethoxysilan, 4-Amino-3-methylbutyltrimethoxysilan, 4-Amino-3-methylbutyltriethoxysilan, 4-Amino-3,3-dimethylbutyltrimethoxysilan, 4-Amino-3,3-dimethylbutyltriethoxysilan, 2-Aminoethyltrimethoxysilan oder 2-Aminoethyltriethoxysilan. Besonders bevorzugt sind 3-Aminopropyltrimethoxysilan, 3-Aminopropyltriethoxysilan, 4-Amino-3,3-dimethylbutyltrimethoxysilan oder 4-Amino-3,3-dimethylbutyltriethoxysilan.
Als Lactone geeignet sind insbesondere γ-Valerolacton, γ-Octalacton, δ-Decalacton oder ε-Decalacton, insbesondere γ-Valerolacton.
Als cyclische Carbonate geeignet sind insbesondere 4,5-Dimethyl-1,3-dioxolan-2-on, 4,4-Dimethyl-1,3-dioxolan-2-on, 4-Ethyl-1,3-dioxolan-2-on, 4-Methyl-1,3-dioxolan-2-on oder 4-(Phenoxymethyl)-1,3-dioxolan-2-on.
Als Lactide geeignet sind insbesondere 1,4-Dioxan-2,5-dion (Lactid aus 2-Hydroxyessigsäure, auch "Glycolid" genannt), 3,6-Dimethyl-1,4-dioxan-2,5-dion (Lactid aus Milchsäure, auch "Lactid" genannt) oder 3,6-Diphenyl-1,4-dioxan-2,5-dion (Lactid aus Mandelsäure).
Bevorzugte Hydroxysilane, welche auf diese Weise erhalten werden, sind N-(3-Triethoxysilylpropyl)-2-hydroxypropanamid, N-(3-Triethoxysilylpropyl)-4-hydroxypentanamid, N-(3-Triethoxysilylpropyl)-4-hydroxyoctanamid, N-(3-Triethoxysilylpropyl)-5-hydroxydecanamid, N-(3-Triethoxysilylpropyl)-2-hydroxypropylcarbamat und die entsprechenden Silane mit Methoxy- anstelle der EthoxyGruppen.

Weiterhin sind geeignete Hydroxysilane auch erhältlich aus der Addition von Aminosilanen an Epoxide oder aus der Addition von Aminen an Epoxysilane. Bevorzugte Hydroxysilane, welche auf diese Weise erhalten werden, sind 2-Morpholino-4(5)-(2-trimethoxysilylethyl)cyclohexan-1-ol, 2-Morpholino-4(5)-(2-triethoxysilylethyl)cyclohexan-1-ol oder 1-Morpholino-3-(3-(triethoxysilyl)propoxy)propan-2-ol.

Als Silangruppen aufweisende Polyether sind auch kommerziell erhältliche Produkte geeignet, insbesondere die Folgenden: MS Polymer™ (von Kaneka Corp.; insbesondere die Typen S203H, S303H, S227, S810, MA903 und S943); MS Polymer™ bzw. Silyl™ (von Kaneka Corp.; insbesondere die Typen SAT010, SAT030, SAT200, SAX350, SAX400, SAX725, MAX450, MAX951); Excestar® (von Asahi Glass Co. Ltd.; insbesondere die Typen S2410, S2420, S3430, S3630); SPUR+* (von Momentive Performance Materials; insbesondere die Typen 1010LM, 1015LM, 1050MM); Vorasil™ (von Dow Chemical Co.; insbesondere die Typen 602 und 604); Desmoseal® (von Covestro AG; insbesondere die Typen S XP 2458, S XP 2636, S XP 2749, S XP 2774 und S XP 2821), TEGOPAC® (von Evonik Industries AG; insbesondere die Typen Seal 100, Bond 150, Bond 250), Polymer ST (von Hanse Chemie AG/Evonik Industries AG, insbesondere die Typen 47, 48, 61, 61LV, 77, 80, 81); Geniosil® STP (von Wacker Chemie AG; insbesondere die Typen E10, E15, E30, E35).

Besonders bevorzugte Silangruppen aufweisende organische Polymere weisen Endgruppen der Formel (VII) auf, 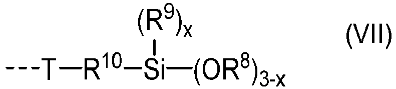 wobei
R¹⁰ für einen zweiwertigen, linearen oder verzweigten Kohlenwasserstoffrest mit 1 bis 12 C-Atomen, welcher gegebenenfalls cyclische und/oder aromatische Anteile und gegebenenfalls ein oder mehrere Heteroatome, insbesondere ein oder mehrere Stickstoffatome, aufweist, steht,
T für einen zweiwertigen Rest ausgewählt aus -O-, -S-, -N(R¹¹)-, -O-CO-N(R¹¹)-, -N(R¹¹)-CO-O- und -N(R¹¹)-CO-N(R¹¹)- steht,
   wobei R¹¹ für einen Wasserstoff-Rest oder für einen linearen oder verzweigten Kohlenwasserstoff-Rest mit 1 bis 20 C-Atomen, welcher gegebenenfalls cyclische Anteile aufweist, und welcher gegebenenfalls eine Alkoxysilan-, Ether- oder Carbonsäureester-Gruppe aufweist, steht, und
R⁸, R⁹ und x die bereits genannten Bedeutungen aufweisen.
Bevorzugt steht R¹⁰ für 1,3-Propylen oder für 1,4-Butylen, wobei Butylen mit einer oder zwei Methylgruppen substituiert sein kann.
Besonders bevorzugt steht R¹⁰ für 1,3-Propylen.

Ein weiterer Gegenstand der Erfindung ist eine härtbare Zusammensetzung umfassend mindestens ein Amidin enthaltend mindestens eine Struktureinheit der Formel (I), wie vorgängig beschrieben. Dabei katalysiert das Amidin die Vernetzung bzw. Aushärtung der Zusammensetzung.

Bevorzugt enthält die härtbare Zusammensetzung Reaktivgruppen ausgewählt aus Silangruppen, Isocyanatgruppen, Epoxidgruppen und Cyanatestergruppen.

Besonders bevorzugt enthält die härtbare Zusammensetzung Silangruppen und/oder Isocyanatgruppen, insbesondere Silangruppen.

Bevorzugt enthält die härtbare Zusammensetzung mindestens ein Polyisocyanat oder mindestens ein Isocyanatgruppen aufweisendes Polyurethanpolymer oder mindestens ein Silan oder mindestens ein Silangruppen aufweisendes Polymer, wie vorgängig beschrieben.

Besonders bevorzugt enthält die härtbare Zusammensetzung mindestens ein Silan und/oder mindestens ein Silangruppen aufweisendes Polymer.

Bevorzugt wird die härtbare Zusammensetzung eingesetzt zum Verkleben, Abdichten, Dämmen, Beschichten oder Vorbehandeln in Bau- und Industrieanwendungen, insbesondere als Betonelementklebstoff, Fassadenklebstoff, Parkettklebstoff, Fensterprofilklebstoff, Verankerungsklebstoff, Montageklebstoff, Karosserieklebstoff, Scheibenklebstoff, Sandwichelementklebstoff, Kaschierklebstoff, Laminatklebstoff, Verpackungsklebstoff, Fugendichtstoff, Bodenfuge, Spachtelmasse, Abdichtungsmembran, Bördel- oder Schweissnahtdichtstoff, Hohlraumversiegelung, Bauschaum, Möbelschaum, Filterschaum, Isolationsschaum, Schalldämmschaum, Verpackungsschaum, Karosserieschaum, Bodenbelag, Bodenbeschichtung, Balkonbeschichtung, Dachbeschichtung, Betonschutzbeschichtung, Parkhausbeschichtung, Rohrbeschichtung, Korrosionsschutzbeschichtung, Textilbeschichtung, Primer, Aktivator oder Haftbrücke, oder als Formteil, Halbzeug, Folie oder Faser, insbesondere als Kissen, Polster, Matratze, Schuhsohle, Stossdämpfer, Dämpfungselement, Dichtung, Reifen, Rolle, Lager, Walze, Förderband, Schlauch, Gehäuse, Fensterprofil, Isolationsplatte, Modellbauplatte, Sandwichelement, Faserverbundkörper, Implantat, Verpackungsfolie, Kaschierfolie oder Textilfaser.

Insbesondere stellt die härtbare Zusammensetzung einen Klebstoff oder einen Dichtstoff oder eine Beschichtung dar.

Am meisten bevorzugt enthält die härtbare Zusammensetzung mindestens ein Silangruppen aufweisendes Polymer, insbesondere ausgewählt aus der Gruppe bestehend aus Polyorganosiloxanen mit endständigen Silangruppen und Silangruppen aufweisenden organischen Polymeren, wie vorgängig beschrieben.
Eine solche Zusammensetzung verfügt über eine gute Lagerstabilität ohne Separationsneigung, erlaubt aufgrund der geringen Toxizität und geringen Flüchtigkeit des Amidins enthaltend mindestens eine Struktureinheit der Formel (I) eine niedrige Gefahreneinstufung und ermöglicht emissionsarme und weitgehend geruchlose Produkte, welche rasch aushärten und dabei ein mechanisch hochwertiges und beständiges Material bilden. Besonders vorteilhaft ist dabei der Umstand, dass dieses Material kaum zu migrationsbedingten Fehlern wie Ausschwitzen oder Substratverschmutzung neigt, im Gegensatz zu Zusammensetzungen mit Katalysatoren nach dem Stand der Technik, wie beispielsweise DBU oder TMG. Zusammensetzungen enthaltend solche aus dem Stand der Technik bekannten Katalysatoren neigen zu Migrationseffekten, was sich vor der Aushärtung durch Separation und nach der Aushärtung durch klebrige und/oder schmierige Oberflächen und/oder Substratverschmutzungen äussern kann. Besonders letztere Effekte sind äusserst unerwünscht, da klebrige und schmierige Oberflächen schnell verschmutzen und schlecht überstreichbar sind, und Substratverunreinigungen zu bleibenden Verfärbungen führen können.

Eine härtbare Zusammensetzung, welche ein Polyorganosiloxan mit endständigen Silangruppen enthält, weist den Vorteil auf, dass sie besonders wasser- und lichtbeständig ist und besonders weichelastische Eigenschaften ermöglicht.

Eine härtbare Zusammensetzung, welche ein Silangruppen aufweisendes organisches Polymer enthält, weist den Vorteil auf, dass sie besonders gute Haftungseigenschaften auf einer Vielzahl von Untergründen aufweist und besonders kostengünstig ist.

Bevorzugt umfasst eine Zusammensetzung, welche ein Polyorganosiloxan mit endständigen Silangruppen, insbesondere ein Polyorganosiloxane der Formel (V), enthält, zusätzlich mindestens einen Silanvernetzer, insbesondere ein Silan der Formel (VIII),

(R¹²)_{q}-Si-(G')_{4-q} (VIII)

wobei
R¹² für einen einwertigen Kohlenwasserstoff-Rest mit 1 bis 12 C-Atomen steht, G' für einen Hydroxyl-Rest oder für einen Alkoxy-, Acetoxy-, Ketoximato-, Amido- oder Enoxy-Rest mit 1 bis 13 C-Atomen steht, und
q für einen Wert von 0, 1 oder 2, insbesondere für 0 oder 1, steht.

Besonders geeignete Silane der Formel (VIII) sind Methyltrimethoxysilan, Ethyltrimethoxysilan, Propytrimethoxysilan, Octyltrimethoxysilan, Isooctyltrimethoxysilan, Vinyltrimethoxysilan, Phenyltrimethoxysilan, Methyltriethoxysilan, Octyltriethoxysilan, Isooctyltriethoxysilan, Vinyltriethoxysilan, Phenyltriethoxysilan, Tetramethoxysilan, Tetraethoxysilan, Methyltris(methylethylketoximo)-silan, Vinyltris(methylethylketoximo)silan oder Methyltris(isobutylketoximo)-silan.

Bevorzugt ist das Amidin enthaltend mindestens eine Struktureinheit der Formel (I) in der härtbaren Zusammensetzung in einer solchen Menge vorhanden, dass die Konzentration an Amidin-Gruppen bezogen auf die Menge der funktionellen Verbindung, insbesondere bezogen auf die Menge des Silangruppen aufweisenden Polymers, im Bereich von 0.1 bis 50 mmol/100 g, bevorzugt 0.2 bis 50 mmol/100 g, insbesondere 0.5 bis 20 mmol/100 g, liegt.
Eine solche Zusammensetzung weist eine gute Lagerfähigkeit und eine schnelle Aushärtung auf.

Zusätzlich zum Amidin enthaltend mindestens eine Struktureinheit der Formel (I) kann die Zusammensetzung weitere Katalysatoren, welche insbesondere die Vernetzung von Isocyanatgruppen und/oder Silangruppen katalysieren, enthalten. Als weitere Katalysatoren geeignet sind insbesondere Metall-Verbindungen und/oder basische Stickstoff- oder Phosphorverbindungen. Geeignete Metall-Verbindungen sind insbesondere Verbindungen von Zinn, Titan, Zirkonium, Aluminium oder Zink, insbesondere Diorganozinn(IV)-Verbindungen wie insbesondere Dibutylzinn(IV)-diacetat, Dibutylzinn(IV)-dilaurat, Dibutylzinn(IV)-dineodecanoat oder Dibutylzinn(IV)-bis(acetylacetonat) und Dioctylzinn(IV)-dilaurat, sowie Titan(IV)- oder Zirkonium(IV)- oder Aluminium(III)- oder Zink(II)-Komplexe mit insbesondere Alkoxy-, Carboxylat-, 1,3-Diketonat-, 1,3-Ketoesterat- oder 1,3-Ketoamidat-Liganden.
Geeignete basische Stickstoff- oder Phosphorverbindungen sind insbesondere Imidazole, Pyridine, Phosphazen-Basen oder bevorzugt Amine, Hexahydrotriazine, Biguanide, Guanidine oder weitere Amidine.
Geeignete Amine sind insbesondere Alkyl-, Cycloalkyl- oder Aralkylamine wie Triethylamin, Triisopropylamin, 1-Butylamin, 2-Butylamin, tert.Butylamin, 3-Methyl-1-butylamin, 3-Methyl-2-butylamin, Dibutylamin, Tributylamin, Hexylamin, Dihexylamin, Cyclohexylamin, Dicyclohexylamin, Dimethylcyclohexylamin, Benzylamin, Dibenzylamin, Dimethylbenzylamin, Octylamin, 2-Ethylhexylamin, Di-(2-ethylhexyl)amin, Laurylamin, N,N-Dimethyl-laurylamin, Stearylamin, N,N-Dimethyl-stearylamin; von natürlichen Fettsäuregemischen abgeleitete Fettamine; aliphatische, cycloaliphatische oder araliphatische Diamine wie Ethylendiamin, Butandiamin, Hexamethylendiamin, Dodecandiamin, Neopentandiamin, 2-Methylpentamethylendiamin (MPMD), 2,2(4),4-Trimethylhexamethylendiamin (TMD), Isophorondiamin (IPD), 2,5(2,6)-Bis-(aminomethyl)-bicyclo[2.2.1]heptan (NBDA), 1,3-Xylylendiamin (MXDA), N,N'-Di(tert.butyl)ethylendiamin, N,N,N',N'-Tetramethylethylendiamin, N,N,N',N'-Tetramethylpropylendiamin, N,N,N',N'-Tetramethylhexamethylendiamin, 3-(Dimethylamino)propylamin, 3-(Methylamino)propylamin, 3-(Cyclohexylamino)propylamin, Piperazin, N-Methylpiperazin, N,N'-Dimethylpiperazin, 1,4-Diazabicyclo[2.2.2]octan (DABCO), Fettpolyamine wie N-Cocoalkyl-1,3-propandiamin; Polyalkylenamine wie Diethylentriamin, Dipropylentriamin, Triethylentetramin (TETA), Tetraethylenpentamin (TEPA), Pentamethylenhexamin (PEHA), 3-(2-Aminoethyl)-aminopropylamin, N,N'-Bis(3-aminopropyl)ethylendiamin, N-(3-Aminopropyl)-N-methylpropandiamin, Bis(3-dimethylaminopropyl)amin, N-(3-Dimethylaminopropyl)-1,3-propylendiamin, N-(2-Aminoethyl)piperazin (N-AEP), N-(2-Aminopropyl)piperazin, N,N'-Di-(2-aminoethyl)piperazin, 1-Methyl-4-(2-dimethylaminoethyl)piperazin, N,N,N',N",N"-Pentamethyldiethylentriamin, N,N,N',N",N"-Pentamethyldipropylentriamin, Polyethylenimine, erhältlich beispielsweise unter den Handelsnamen Lupasol® (von BASF) und Epomin® (von Nippon Shokubai); Etheramine wie insbesondere 2-Methoxyethylamin, 2-Ethoxyethylamin, 3-Methoxypropylamin, 3-Ethoxypropylamin, 3-(2-Ethylhexyloxy)propylamin, 3-(2-Methoxyethoxy)propylamin, Morpholin, N-Methylmorpholin, N-Ethylmorpholin, 2-Aminoethylmorpholin, Bis(2-aminoethyl)ether, Bis(dimethylaminoethyl)ether, Bis(morpholinoethyl)ether (DMDEE), N,N,N'-Trimethyl-N'-hydroxyethylbis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin oder 2-Aminopropylterminierte Glykole, wie sie beispielsweise unter dem Handelsnamen Jeffamin® (von Huntsman) erhältlich sind; Aminoalkohole, wie insbesondere Ethanolamin, Isopropanolamin, Diethanolamin, Diisopopanolamin, Triethanolamin, Triisopropanolamin, N-Butylethanolamin, Diglykolamin, N,N-Diethylethanolamin, N-Methyldiethanolamin, N-Methyldiisopropylamin, N,N,N'-Trimethylaminoethyl-ethanolamin, N-(3-Dimethylaminopropyl)-N,N-diisopropanolamin, N,N-Bis(3-dimethylaminopropyl)-N-isopropanolamin, 2-(2-Dimethylaminoethoxy)ethanolamin oder Addukte aus Mono- und Polyaminen mit Epoxiden oder Diepoxiden; Phenolgruppen-haltige Amine, wie insbesondere Kondensationsprodukte aus Phenolen, Aldehyden und Aminen (sogenannte Mannich-Basen und Phenalkamine) wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin sowie im Handel unter den Markennamen Cardolite® (von Cardolite), Aradur® (von Huntsman) und Beckopox® (von Cytec) erhältliche Phenalkamine; Amidgruppen-haltige Polyamine, sogenannte Polyamidoamine, wie sie im Handel erhältlich sind, beispielsweise unter den Markennamen Versamid® (von Cognis), Aradur® (von Huntsman), Euretek® (von Huntsman) oder Beckopox® (von Cytec); oder Aminosilane, wie insbesondere 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropyl-methyldimethoxysilan, N-(2-Aminoethyl)-N'-[3-(trimethoxysilyl)propyl]ethylendiamin oder deren Analoga mit Ethoxy- anstelle der Methoxygruppen am Siliciumatom.
Geeignete Hexahydrotriazine sind insbesondere 1,3,5-Hexahydrotriazin, 1,3,5-Trimethylhexahydrotriazin oder 1,3,5-Tris(3-(dimethylamino)propyl)-hexahydrotriazin.
Geeignete Biguanide sind insbesondere Biguanid, 1-Butylbiguanid, 1,1-Dimethylbiguanid, 1-Butylbiguanid, 1-Phenylbiguanid oder 1-(o-Tolyl)biguanid (OTBG).
Geeignete Guanidine sind insbesondere 1-Butylguanidin, 1,1-Dimethylguanidin, 1,3-Dimethylguanidin, 1,1,3,3-Tetramethylguanidin (TMG), 2-(3-(Trimethoxysilyl)propyl)-1,1,3,3-tetramethylguanidin, 2-(3-(Methyldimethoxysilyl)-propyl)-1,1,3,3-tetramethylguanidin, 2-(3-(Triethoxysilyl)propyl)-1,1,3,3-tetramethylguanidin, 1,5,7-Triazabicyclo[4.4.0]dec-5-en (TBD), 7-Methyl-1,5,7-triazabicyclo[4.4.0]dec-5-en, 7-Cyclohexyl-1,5,7-triazabicyclo[4.4.0]dec-5-en, 1-Phenylguanidin, 1-(o-Tolyl)guanidin (OTG), 1,3-Diphenylguanidin, 1,3-Di(o-tolyl)guanidin oder 2-Guanidinobenzimidazol.

Geeignete weitere Amidine sind insbesondere 1,8-Diazabicyclo[5.4.0]undec-7-en (DBU), 1,5-Diazabicyclo[4.3.0]non-5-en (DBN), 6-Dibutylamino-1,8-diazabicyclo[5.4.0]undec-7-en, 6-Dibutylamino-1,8-diazabicyclo[5.4.0]undec-7-en, N,N'-Di-n-hexylacetamidin (DHA), 2-Methyl-1,4,5,6-tetrahydropyrimidin, 1,2-Dimethyl-1,4,5,6-tetrahydropyrimidin, 2,5,5-Trimethyl-1,4,5,6-tetrahydropyrimidin, N-(3-Trimethoxysilylpropyl)-4,5-dihydroimidazol oder N-(3-Triethoxysilylpropyl)-4,5-dihydroimidazol.

Weiterhin kann die Zusammensetzung als Co-Katalysator eine Säure enthalten, insbesondere eine Carbonsäure. Bevorzugt sind aliphatische Carbonsäuren wie Ameisensäure, Laurinsäure, Stearinsäure, Isostearinsäure, Ölsäure, 2-Ethyl-2,5-dimethylcapronsäure, 2-Ethylhexansäure, Neodecansäure, Fettsäuregemische aus der Verseifung von natürlichen Fetten und Ölen oder Di- und Polycarbonsäuren, insbesondere Poly(meth)acrylsäuren.

Die Zusammensetzung ist in einer bevorzugten Ausführungsform im Wesentlichen frei von Organozinn-Verbindungen. Organozinn-freie Zusammensetzungen sind hinsichtlich Gesundheitsschutz und Umweltschutz vorteilhaft. Insbesondere beträgt der Zinngehalt der härtbaren Zusammensetzung weniger als 0.1 Gew.-%, insbesondere weniger als 0.05 Gew.-%.

Die Zusammensetzung enthält in einer weiteren bevorzugten Ausführungsform eine Kombination aus mindestens einem Amidin enthaltend mindestens eine Struktureinheit der Formel (I) und mindestens einer Organozinn-Verbindung, insbesondere einer Diorganozinn(IV)-Verbindung wie die vorgängig genannten. Eine solche Zusammensetzung weist bereits bei einem geringen Zinn-Gehalt eine hohe Aushärtungsgeschwindigkeit auf, was aus toxikologischen und ökologischen Gründen vorteilhaft ist.

In einer Ausführungsform enthält die Zusammensetzung neben dem Amidin enthaltend mindestens eine Struktureinheit der Formel (I) zusätzlich mindestens ein Organotitanat. Eine Kombination aus dem Amidin enthaltend mindestens eine Struktureinheit der Formel (I) und einem Organotitanat weist eine besonders hohe katalytische Aktivität auf. Dadurch wird eine schnelle Aushärtung bei einer verhältnismässig geringen Einsatzmenge an Organotitanat ermöglicht.

Als Organotitanat geeignet sind insbesondere Titan(IV)-Komplexverbindungen. Bevorzugte Organoititanate sind insbesondere ausgewählt aus
- Titan(IV)-Komplexverbindungen mit zwei 1,3-Diketonat-Liganden, insbesondere 2,4-Pentandionat (=Acetylacetonat), und zwei Alkoholat-Liganden;
- Titan(IV)-Komplexverbindungen mit zwei 1,3-Ketoesterat-Liganden, insbesondere Ethylacetoacetat, und zwei Alkoholat-Liganden;
- Titan(IV)-Komplexverbindungen mit einem oder mehreren Aminoalkoholat-Liganden, insbesondere Triethanolamin oder 2-((2-Aminoethyl)amino)ethanol, und einem oder mehreren Alkoholat-Liganden;
- Titan(IV)-Komplexverbindungen mit vier Alkoholat-Liganden (Orthotitanate);
- sowie höherkondensierte Organotitanate, insbesondere oligomeres Titan(IV)-tetrabutanolat, auch als Polybutyltitanat bezeichnet;
wobei als Alkoholat-Liganden insbesondere Isobutoxy, n-Butoxy, Isopropoxy, Ethoxy und 2-Ethylhexoxy geeignet sind.

Insbesondere geeignet sind die kommerziell erhältlichen Typen Tyzor® AA, GBA, GBO, AA-75, AA-65, AA-105, DC, BEAT, BTP, TE, TnBT, KTM, TOT, TPT oder IBAY (alle von Dorf Ketal); Tytan PBT, TET, X85, TAA, ET, S2, S4 oder S6 (alle von Borica Company Ltd.) und Ken-React® KR® TTS, 7, 9QS, 12, 26S, 33DS, 38S, 39DS, 44, 134S, 138S, 133DS, 158FS oder LICA® 44 (alle von Kenrich Petrochemicals).

Ganz besonders geeignete Organotitanate sind ausgewählt aus Bis(ethylacetoacetato)diisobutoxy-titan(IV) (kommerziell erhältlich beispielsweise als Tyzor® IBAY von Dorf Ketal), Bis(ethylacetoacetato)diisopropoxy-titan(IV) (kommerziell erhältlich beispielsweise als Tyzor® DC von Dorf Ketal), Bis(acetylacetonato)diisopropoxy-titan(IV), Bis(acetylacetonato)diisobutoxy-titan(IV), Tris(oxyethyl)-amin-isopropoxy-titan(IV), Bis[tris(oxyethyl)amin]diisopropoxy-titan(IV), Bis(2-ethylhexan-1,3-dioxy)-titan(IV), Tris[2-((2-Aminoethyl)amino)ethoxy]ethoxy-titan(IV), Bis(neopentyl(diallyl)oxy)-diethoxy-titan(IV), Tetra(isopropoxy)titanat, Tetra(n-butoxy)titanat, Tetra(2-ethylhexyloxy)titanat und Polybutyltitanat. Am meisten bevorzugt sind Bis(ethylacetoacetato)diisobutoxy-titan(IV) oder Bis(ethylacetoacetato)diisopropoxy-titan(IV).

Die härtbare Zusammensetzung enthält bevorzugt mindestens einen weiteren Bestandteil ausgewählt aus der Gruppe bestehend aus Füllstoffen, Weichmachern, Rheologie-Additiven, Trocknungsmitteln, Haftvermittlern und Vernetzern. Besonders bevorzugt enthält sie eine beliebige Kombination von mehreren dieser Bestandteile.

Geeignete Füllstoffe sind insbesondere anorganische oder organische Füllstoffe, insbesondere natürliche, gemahlene oder gefällte Calciumcarbonate, welche gegebenenfalls mit Fettsäuren, insbesondere Stearinsäure, beschichtet sind, Baryt (Schwerspat), Talke, Quarzmehle, Quarzsand, Dolomite, Wollastonite, Kaoline, calcinierte Kaoline, Mica (Kalium-Aluminium-Silikat), Molekularsiebe, Aluminiumoxide, Aluminiumhydroxide, Magnesiumhydroxid, Kieselsäuren inklusive hochdisperse Kieselsäuren aus Pyrolyseprozessen, industriell hergestellte Russe, Graphit, Metall-Pulver wie Aluminium, Kupfer, Eisen, Silber oder Stahl, PVC-Pulver oder Hohlkugeln.

Geeignete Weichmacher sind insbesondere Trialkylsilyl-terminierte Polydialkylsiloxane, vorzugsweise Trimethylsilyl-terminierte Polydimethylsiloxane, insbesondere mit Viskositäten im Bereich von 10 bis 1'000 mPa·s, oder entsprechende Verbindungen, bei denen einige der Methylgruppen ersetzt sind durch andere organische Gruppen, insbesondere Phenyl-, Vinyl- oder Trifluorpropylgruppen, sogenannte Reaktivweichmacher in Form von monofunktionellen, also einseitig reaktiven, Polysiloxanen, Carbonsäureester wie Phthalate, insbesondere Dioctylphthalat, Bis(2-ethylhexyl)phthalat, Bis(3-propylheptyl)phthalat, Diisononylphthalat oder Diisodecylphthalat, Diester von ortho-Cyclohexandicarbonsäure, insbesondere Diisononyl-1,2-cyclohexandicarboxylat, Adipate, insbesondere Dioctyladipat, Bis(2-Ethylhexyl)adipat, Azelate, insbesondere Bis(2-ethylhexyl)acelat, Sebacate, insbesondere Bis(2-ethylhexyl)sebacat oder Diisononylsebacat, Polyole, insbesondere Polyoxyalkylenpolyole oder Polyesterpolyole, Glykolether, Glykolester, organische Phosphor- oder Sulfonsäureester, Sulfonsäureamide, Polybutene oder von natürlichen Fetten oder Ölen abgeleitete Fettsäuremethyl- oder -ethylester, auch "Biodiesel" genannt, wobei Siloxangruppen-haltige Weichmacher besonders geeignet sind für Silangruppen aufweisende Polymere in Form von Polyorganosiloxanen.

Geeignete Rheologie-Additive sind insbesondere Verdickungsmittel, insbesondere Schichtsilikate wie Bentonite, Derivate von Rizinusöl, hydriertes Rizinusöl, Polyamide, Polyurethane, Harnstoffverbindungen, pyrogene Kieselsäuren, Celluloseether oder hydrophob modifizierte Polyoxyethylene.

Geeignete Trocknungsmittel sind insbesondere Tetraethoxysilan, Vinyltrimethoxysilan, Vinyltriethoxysilan oder Organoalkoxysilane, welche in α-Stellung zur Silangruppe eine funktionelle Gruppe aufweisen, insbesondere N-(Methyldimethoxysilylmethyl)-O-methyl-carbamat, (Methacryloxymethyl)silane, Methoxymethylsilane, Orthoameisensäureester, Calciumoxid, Molekularsiebe, hochreaktive Isocyanate wie p-Tosylisocyanat, monomere Diisocyanate oder Mono-Oxazolidine wie Incozol® 2 (von Incorez), insbesondere Vinyltrimethoxysilan oder Vinyltriethoxysilan.

Geeignete Haftvermittler und/oder Vernetzer sind insbesondere Aminosilane wie insbesondere 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropyldimethoxymethylsilan, N-(2-Aminoethyl)-N'-[3-(trimethoxysilyl)propyl]ethylendiamin oder deren Analoga mit Ethoxy- anstelle von Methoxygruppen, weiterhin N-Phenyl-, N-Cyclohexyl- oder N-Alkylaminosilane, Mercaptosilane, Epoxysilane, (Meth)acrylosilane, Anhydridosilane, Carbamatosilane, Alkylsilane oder Iminosilane, oligomere Formen dieser Silane, Addukte aus primären Aminosilanen mit Epoxysilanen oder (Meth)acrylosilanen oder Anhydridosilanen, aminofunktionelle Alkylsilsesquioxane, insbesondere aminofunktionelles Methylsilsesquioxan oder aminofunktionelles Propylsilsesquioxan. Insbesondere geeignet sind 3-Aminopropyltrimethoxysilan, 3-Aminopropyltriethoxysilan, N-(2-Aminoethyl)-3-aminopropyltrimethoxysilan, N-(2-Aminoethyl)-3-aminopropyltriethoxysilan, 3-Glycidoxypropyltrimethoxysilan, 3-Glycidoxypropyltriethoxysilan oder 3-Ureidopropyltrimethoxysilan, oder oligomere Formen dieser Silane.

In einer bevorzugten Ausführungsform enthält die Zusammensetzung mindestens ein Trocknungsmittel und mindestens einen Haftvermittler und/oder Vernetzer.

In einer bevorzugten Ausführungsform enthält die Zusammensetzung keine Phthalate als Weichmacher. Solche Zusammensetzungen sind toxikologisch vorteilhaft und weisen gegebenenfalls weniger Probleme mit Migrationseffekten auf.

Für den Fall, dass die Zusammensetzung ein Polyisocyanat und/oder ein Isocyanatgruppen aufweisendes Polyurethanpolymer enthält, ist zusätzlich bevorzugt mindestens eine gegenüber Isocyanatgruppen reaktive mehrfunktionelle Verbindung vorhanden, wie insbesondere
- ein oder mehrere Polyole, insbesondere die als geeignet für die Herstellung eines Isocyanatgruppen aufweisendes Polyurethanpolymers genannten Polyole. Bevorzugt sind Polyetherpolyole, Polyesterpolyole, Polycarbonatpolyole, Poly(meth)acrylatpolyole oder Polybutadienpolyole. Besonders bevorzugt sind Polyetherpolyole, insbesondere Polyoxypropylenpolyole und/oder Ethylenoxid-terminierte Polyoxypropylenpolyole. Bevorzugt sind Polyole mit einem mittleren Molekulargewicht im Bereich von 400 bis 10'000 g/mol, insbesondere 500 bis 6'000 g/mol. Bevorzugt sind Polyole mit einer mittleren OH-Funktionalität im Bereich von 1.6 bis 4, insbesondere 1.8 bis 3, besonders bevorzugt 2.2 bis 3. Ebenfalls geeignet sind Polyetherpolyole mit darin dispergierten Polymerpartikeln, insbesondere solche mit Styrol-Acrylnitril-Partikeln (SAN) oder Polyharnstoff- bzw. Polyhydrazodicarbonamid-Partikeln (PHD).
- Kettenverlängerer, insbesondere 1,2-Ethandiol, 1,3-Propandiol, 2-Methyl-1,3-propandiol, 1,4-Butandiol, 1,5-Pentandiol, Neopentylglykol, 1,6-Hexandiol, 3-Methyl-1,5-pentandiol, 1,7-Heptandiol, 1,8-Octandiol, 1,3-Cyclohexandimethanol, 1,4-Cyclohexandimethanol, Diethylenglykol oder Triethylenglykol;
- Aminoalkohole, insbesondere 2-Aminoethanol, 2-(2-Aminoethoxy)ethanol oder 3-Aminomethyl-3,5,5-trimethylcyclohexanol oder Ether-, Ester- oder Urethangruppen aufweisende Derivate davon;
- blockierte Aminogruppen aufweisende Verbindungen, insbesondere Aldimine, Ketimine, Enamine, Oxazolidine, Imidazolidine oder Hexahydropyrimidine.;
- oder Polyamine.

Die Zusammensetzung kann weitere Bestandteile enthalten, insbesondere die folgenden Hilfs- und Zusatzmittel:
- Pigmente, insbesondere Titandioxid oder Eisenoxide;
- Farbstoffe;
- Stabilisatoren gegen Oxidation, Wärme, Licht oder UV-Strahlung;
- natürliche Harze, Fette oder Öle wie Kolophonium, Schellack, Leinöl, Rizinusöl oder Sojaöl;
- nicht-reaktive Polymere, wie insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid und Magnesiumhydroxid, oder insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)-phosphat, isopropyliertes Triphenylphosphat, Mono-, Bis- oder Tris(isopropylphenyl)phosphate unterschiedlichen Isopropylierungsgrades, Resorcinolbis(diphenylphosphat), Bisphenol-A-bis(diphenylphosphat) oder Ammoniumpolyphosphate;
- Fasern, insbesondere Glasfasern, Kohlefasern, Metallfasern, Keramikfasern oder Kunststofffasern wie Polyamidfasern oder Polyethylenfasern;
- oberflächenaktive Substanzen, insbesondere Netzmittel, Verlaufsmittel, Entlüftungsmittel oder Entschäumer;
- Lösemittel;
- Biozide, insbesondere Algizide, Fungizide oder das Pilzwachstum hemmende Substanzen;
sowie weitere üblicherweise in härtbaren Zusammensetzungen eingesetzte Substanzen.
Es kann sinnvoll sein, gewisse Bestandteile vor dem Einmischen in die Zusammensetzung chemisch oder physikalisch zu trocknen.

Die Zusammensetzung wird vorzugsweise unter Ausschluss von Feuchtigkeit hergestellt und aufbewahrt. Typischerweise ist sie in einer geeigneten Verpackung oder Anordnung, wie insbesondere einer Kartusche, einer Flasche, einer Büchse, einem Beutel, einem Eimer, einem Hobbock oder einem Fass, unter Ausschluss von Feuchtigkeit lagerstabil.

Die Zusammensetzung kann in Form einer einkomponentigen oder in Form einer mehrkomponentigen, insbesondere zweikomponentigen, Zusammensetzung vorliegen.
Als "einkomponentig" wird im vorliegenden Dokument eine Zusammensetzung bezeichnet, bei welcher alle Bestandteile der Zusammensetzung vermischt im gleichen Gebinde gelagert werden und welche mit Feuchtigkeit härtbar ist. Als "zweikomponentig" wird im vorliegenden Dokument eine Zusammensetzung bezeichnet, bei welcher die Bestandteile der Zusammensetzung in zwei verschiedenen Komponenten vorliegen, welche in voneinander getrennten Gebinden gelagert werden. Erst kurz vor oder während der Applikation der Zusammensetzung werden die beiden Komponenten miteinander vermischt, worauf die vermischte Zusammensetzung aushärtet, gegebenenfalls unter Einwirkung von Feuchtigkeit.

Für den Fall, dass die Zusammensetzung ein Polyisocyanat und/oder ein Isocyanatgruppen aufweisendes Polyurethanpolymer enthält, ist sie bevorzugt zweikomponentig. Dabei enthält die eine Komponente das Polyisocyanat und/oder das Isocyanatgruppen aufweisende Polyurethanpolymer und die andere Komponente enthält das Amidin enthaltend mindestens eine Struktureinheit der Formel (I) und zusätzlich mindestens eine gegenüber Isocyanatgruppen reaktive mehrfunktionelle Verbindung.

Für den Fall, dass die Zusammensetzung ein Silangruppen aufweisendes organisches Polymer enthält, ist sie bevorzugt einkomponentig.

Für den Fall, dass die Zusammensetzung ein Polyorganosiloxan mit endständigen Silangruppen enthält, ist sie bevorzugt einkomponentig, auch als RTV-1 bezeichnet, oder zweikomponentig, auch als RTV-2 bezeichnet. Im Fall einer RTV-2 Zusammensetzung ist das Polyorganosiloxan mit endständigen Silangruppen bevorzugt ein Bestandteil der ersten Komponente und ein Silanvernetzer, insbesondere ein Silan der Formel (VIII), ist bevorzugt ein Bestandteil der zweiten Komponente. Das Amidin enthaltend mindestens eine Struktureinheit der Formel (I) kann dabei in der ersten und/oder in der zweiten Komponente enthalten sein.

Eine zweite oder gegebenenfalls weitere Komponenten wird bzw. werden mit der ersten Komponente vor oder bei der Applikation vermischt, insbesondere über einen Statikmischer oder über einen dynamischen Mischer.
Die Zusammensetzung wird insbesondere umgebungswarm, bevorzugt in einem Temperaturbereich zwischen 0°C und 45°C, insbesondere 5°C bis 35°C, appliziert und härtet auch bei diesen Bedingungen aus.

Bei der Applikation beginnt die Vernetzungsreaktion der funktionellen Gruppen, gegebenenfalls unter Einfluss von Feuchtigkeit.
Vorhandene Isocyanatgruppen reagieren mit Hydroxylgruppen, oder primären oder sekundären Aminogruppen, oder unter dem Einfluss von Feuchtigkeit mit blockierten Aminogruppen. Gegebenenfalls vorhandene weitere Isocyanatgruppen reagieren unter dem Einfluss von Feuchtigkeit untereinander. Vorhandene Silangruppen können mit vorhandenen Silanolgruppen zu Siloxangruppen (Si-O-Si-Gruppen) kondensieren. Vorhandene Silangruppen können bei Kontakt mit Feuchtigkeit auch zu Silanolgruppen (Si-OH-Gruppen) hydrolysieren und durch nachfolgende Kondensationsreaktionen Siloxangruppen (Si-O-Si-Gruppen) bilden.
Als Ergebnis dieser Reaktionen härtet die Zusammensetzung schliesslich aus. Das Amidin enthaltend mindestens eine Struktureinheit der Formel (I) beschleunigt diese Aushärtung.
Falls für die Aushärtung Wasser benötigt wird, kann dieses entweder aus der Luft stammen (Luftfeuchtigkeit), oder aber die Zusammensetzung kann mit einer Wasser enthaltenden Komponente in Kontakt gebracht werden, zum Beispiel durch Bestreichen, beispielsweise mit einem Abglättmittel, oder durch Besprühen, oder es kann der Zusammensetzung bei der Applikation Wasser oder eine Wasser enthaltende Komponente zugesetzt werden, zum Beispiel in Form einer wasserhaltigen oder Wasser freisetzenden Flüssigkeit oder Paste. Eine Paste ist insbesondere geeignet für den Fall, dass die Zusammensetzung selber in Form einer Paste vorliegt.
Bei einer Aushärtung mittels Luftfeuchtigkeit härtet die Zusammensetzung von aussen nach innen durch, wobei zunächst eine Haut an der Oberfläche der Zusammensetzung gebildet wird. Die sogenannte Hautbildungszeit stellt ein Mass für die Aushärtungsgeschwindigkeit der Zusammensetzung dar. Die Geschwindigkeit der Aushärtung wird dabei im Allgemeinen von verschiedenen Faktoren, wie beispielsweise der Verfügbarkeit von Wasser, der Temperatur usw., bestimmt.

Die Zusammensetzung eignet sich für eine Vielzahl von Anwendungen, insbesondere als Anstrich, Lack oder Primer, als Harz zur Herstellung von Faserverbundwerkstoff (Composite), als Hartschaum, Weichschaum, Formteil, Elastomer, Faser, Folie oder Membran, als Vergussmasse, Dichtstoff, Klebstoff, Belag, Beschichtung oder Anstrich für Bau- und Industrieanwendungen, beispielsweise als Nahtabdichtung, Hohlraumversiegelung, Elektroisolationsmasse, Spachtelmasse, Fugendichtstoff, Schweiss- oder Bördelnahtdichtstoff, Montageklebstoff, Karrosserieklebstoff, Scheibenklebstoff, Sandwichelementklebstoff, Kaschierklebstoff, Laminatklebstoff, Verpackungsklebstoff, Holzklebstoff, Parkettklebstoff, Verankerungsklebstoff, Bodenbelag, Bodenbeschichtung, Balkonbeschichtung, Dachbeschichtung, Betonschutzbeschichtung, Parkhausbeschichtung, Versiegelung, Rohrbeschichtung, Korrosionsschutzbeschichtung, Textilbeschichtung, Dämpfungselement, Dichtungselement oder Spachtelmasse.

Besonders geeignet ist die Zusammensetzung als Klebstoff und/oder Dichtstoff, insbesondere für die Fugenabdichtung und für elastische Klebeverbindungen in Bau- und Industrieanwendungen, oder als elastische Beschichtung mit rissüberbrückenden Eigenschaften, insbesondere zum Schützen und/oder Abdichten von beispielsweise Dächern, Böden, Balkonen, Parkdecks oder Betonröhren.

Bevorzugt stellt die Zusammensetzung somit einen Klebstoff oder einen Dichtstoff oder eine Beschichtung dar.
Eine solche Zusammensetzung enthält typischerweise Füllstoffe, Weichmacher, Trocknungsmittel, Haftvermittler und/oder Vernetzer und gegebenenfalls weitere Hilfs- und Zusatzstoffe.
Für eine Anwendung als Klebstoff oder Dichtstoff weist die Zusammensetzung bevorzugt eine pastöse Konsistenz mit strukturviskosen Eigenschaften auf. Ein solcher pastöser Dichtstoff oder Klebstoff wird insbesondere aus handelsüblichen Kartuschen, welche manuell, mittels Druckluft oder Batterie betrieben werden, oder aus einem Fass oder Hobbock mittels einer Förderpumpe oder eines Extruders, gegebenenfalls mittels eines Applikationsroboters, auf ein Substrat aufgetragen.
Für eine Anwendung als Beschichtung weist die Zusammensetzung bevorzugt eine bei Raumtemperatur flüssige Konsistenz mit selbstverlaufenden Eigenschaften auf. Gegebenenfalls ist sie leicht thixotrop, so dass die Beschichtung an geneigten bis senkrechten Flächen applizierbar ist, ohne sofort wegzufliessen. Sie wird insbesondere appliziert mittels Rolle oder Pinsel oder durch Ausgiessen und Verteilen mittels beispielsweise einem Roller, einem Schaber oder einer Zahntraufel.

Bei der Applikation wird die Zusammensetzung bevorzugt auf mindestens ein Substrat appliziert.
Geeignete Substrate sind insbesondere
- Glas, Glaskeramik, Beton, Mörtel, Backstein, Ziegel, Gips oder Natursteine wie Kalkstein, Granit oder Marmor;
- Metalle und Legierungen, wie Aluminium, Eisen, Stahl oder Buntmetalle, sowie oberflächenveredelte Metalle oder Legierungen, wie verzinkte oder verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe und weitere sogenannte Polymer-Composites;
- Kunststoffe, wie Polyvinylchlorid (Hart- und Weich-PVC), Acrylonitril-Butadien-Styrol-Copolymere (ABS), Polycarbonat (PC), Polyamid (PA), Polyester, Poly(methylmethacrylat) (PMMA), Epoxidharze, Polyurethane (PUR), Polyoxymethylen (POM), Polyolefine (PO), Polyethylen (PE) oder Polypropylen (PP), Ethylen/Propylen-Copolymere (EPM) oder Ethylen/Propylen/Dien-Terpolymere (EPDM), oder faserverstärkte Kunststoffe wie Kohlefaserverstärkte Composite-Kunststoffe (CFK), Glasfaser-verstärkte Kunststoffe (GFK) oder Sheet Moulding Compounds (SMC), wobei die Kunststoffe bevorzugt mittels Plasma, Corona oder Flammen oberflächenbehandelt sein können;
- beschichtete Substrate, wie pulverbeschichtete Metalle;
- Farben oder Lacke, insbesondere Automobildecklacke, Metalllacke, Möbellacke oder Holzlacke.
Die Substrate können bei Bedarf vor dem Applizieren der Zusammensetzung vorbehandelt werden, insbesondere durch physikalische und/oder chemische Reinigungsverfahren oder durch das Aufbringen eines Haftvermittlers, einer Haftvermittlerlösung oder eines Primers.
Besonders geeignet ist die Zusammensetzung für den Kontakt mit Substraten, die besonders empfindlich sind auf Störungen durch migrierende Substanzen, insbesondere durch das Ausbilden von Verfärbungen oder Flecken. Dies sind insbesondere feinporige Substrate wie Marmor, Kalkstein oder andere Natursteine, Gips, Zementmörtel oder Beton, aber auch Kunststoffe. Insbesondere auf PVC werden bei der Anwesenheit von Katalysatoren wie beispielsweise DBU oder TMG starke Verfärbungen beobachtet, die sich durch Reinigung nicht entfernen lassen. Solche Effekte werden mit dem Amidin enthaltend mindestens eine Struktureinheit der Formel (I) nicht beobachtet.
Verklebt oder abgedichtet werden können zwei gleichartige oder zwei verschiedene Substrate, insbesondere die vorgängig genannten Substrate.

Nach der Aushärtung der Zusammensetzung wird eine ausgehärtete Zusammensetzung erhalten.
Ein weiterer Gegenstand der Erfindung ist somit eine ausgehärtete Zusammensetzung, erhalten aus der beschriebenen härtbaren Zusammensetzung.

Aus der Anwendung der Zusammensetzung entsteht ein Artikel, welcher mit der Zusammensetzung insbesondere verklebt, abgedichtet oder beschichtet wurde. Beim Artikel handelt es sich insbesondere um ein Bauwerk, insbesondere ein Bauwerk des Hoch- oder Tiefbaus, um ein industriell gefertigtes Gut oder um ein Konsumgut, insbesondere ein Fenster, eine Haushaltmaschine oder ein Transportmittel wie insbesondere ein Automobil, ein Bus, ein Lastkraftwagen, ein Schienenfahrzeug, ein Schiff, ein Flugzeug oder ein Helikopter; oder der Artikel kann ein Anbauteil davon sein.

Die härtbare Zusammensetzung ist lagerfähig und dank geringem Geruch angenehm applizierbar. Nach der Applikation baut sie überraschend schnell Festigkeit auf, wobei mechanisch hochwertige und beständige Materialien entstehen. Die Zusammensetzung neigt weder vor noch nach der Aushärtung zu migrationsbedingten Fehlern wie Separation, Ausschwitzen oder Substratverschmutzung.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.
Als "Normklima" wird eine Temperatur von 23±1°C und eine relative Luftfeuchtigkeit von 50±5% bezeichnet.
Die verwendeten Chemikalien waren, sofern nicht anders bezeichnet, von Sigma-Aldrich.
**¹H-NMR-Spektren** wurden auf einem Spektrometer des Typs Bruker Ascend 400 bei 400.14 MHz gemessen; die chemischen Verschiebungen δ sind angegeben in ppm relativ zu Tetramethylsilan (TMS). Echte und Pseudo-Kopplungsmuster wurden nicht unterschieden.
**Infrarotspektren** (FT-IR) wurden auf einem mit horizontaler ATR-Messeinheit mit Diamant-Kristall ausgestatteten FT-IR Gerät Nicolet iS5 von Thermo Scientific gemessen. Flüssige Proben wurden unverdünnt als Filme aufgetragen, feste Proben wurden in CH₂Cl₂ gelöst. Die Absorptionsbanden sind in Wellenzahlen (cm⁻¹) angegeben (Messfenster: 4000-650 cm⁻¹).
Die **Hautbildungszeit** (HBZ) wurde dadurch bestimmt, dass einige Gramm der Zusammensetzung in einer Schichtdicke von ca. 2 mm auf Pappkarton aufgetragen wurden und im Normklima die Zeitdauer gemessen wurde, bis beim leichten Antippen der Oberfläche der Zusammensetzung mittels einer Pipette aus LDPE erstmals keine Rückstände auf der Pipette mehr zurückblieben.
Die Beschaffenheit der **Oberfläche** wurde haptisch geprüft.
Die mechanischen Eigenschaften **Zugfestigkeit, Bruchdehnung** und **E-Modul** (bei 0-5% und 0-50% Dehnung) wurden gemäss DIN EN 53504 bei einer Zuggeschwindigkeit von 200 mm/min. gemessen.
Die **Viskosität** wurde auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 U/min) gemessen.

### Herstellung von Verbindungen der Formel (IV):

### N,N-Diethyl-(2-oxocyclopentyl)carbonamid:

N,N-Diethyl-(2-oxocyclopentyl)carbonamid wurde hergestellt durch Umsetzung von 2-Oxocyclopentancarbonsäure-methylester mit Diethylamin unter Entfernung von Methanol und nachfolgender Reinigung mittels Destillation. Erhalten wurde eine leicht gelbliche Flüssigkeit mit einer Siedetemperatur von 110°C bei 0.1 mbar.
FT-IR: 2967, 2933, 2877, 1737, 1628, 1447, 1431, 1380, 1361, 1327, 1262, 1219, 1139, 1099, 1003, 973, 921, 835, 820, 786, 682.

### N,N-Dibutyl-(2-oxocyclopentyl)carbonamid:

N,N-Dibutyl-(2-oxocyclopentyl)carbonamid wurde hergestellt durch Umsetzung von 2-Oxocyclopentancarbonsäure-methylester mit Dibutylamin unter Entfernung von Methanol und nachfolgender Reinigung mittels Destillation. Erhalten wurde eine leicht gelbliche Flüssigkeit mit einer Siedetemperatur von 130°C bei 0.1 mbar.
FT-IR: 2957, 2931, 2872, 1740, 1671, 1634, 1564, 1445, 1427, 1373, 1295, 1253, 1181, 1140, 1104, 1053, 1003, 928, 903, 834, 767, 732.

### Herstellung von Amidinen:

**Amidin A1:** 5-(1-(3-Dimethylaminopropyl)-1,4,5,6-tetrahydropyrimidin-2-yl)-N,N-diethylpentanamid
In einem Rundkolben wurden 17.6 g (0.11 mol) 3-(3-(Dimethylamino)propylamino)propylamin (von BASF) in 30 ml Toluol vorgelegt und unter Rühren und Kühlen langsam 20.3 g (0.11 mol) N,N-Diethyl-(2-oxocyclopentyl)carbonamid, hergestellt wie vorgängig beschrieben, zugetropft. Die Reaktionsmischung wurde über Nacht bei 40 °C gerührt. Anschliessend wurde das Azeotrop aus Toluol und Wasser bei 40 °C und 10 mbar abdestilliert. Dann wurde bei Normaldruck auf 135 bis 140 °C erhitzt und das restliche Toluol destillativ entfernt. Nach Abkühlen auf Raumtemperatur wurden 32.5 g Produkt als hellorange, geruchsfreie, niedrigviskose Flüssigkeit erhalten.
¹H-NMR (CDCl₃): 1.03 und 1.07 (2x t, 6 H, J= 7.1 Hz, N(CH₂C*H*₃)₂), 1.55 bis 1.64 (m, 6 H, CH₂C*H*₂C*H*₂CH₂CO und CH₂C*H*₂CH₂N(CH₃)₂), 1.72 (p, 2 H, CH=NCH₂C*H*₂CH₂N), 2.14 (s, 6 H, N(CH₃)₂), 2.17 (m, 4 H, N=CHC*H*₂ und CH₂CO), 2.26 (m, 2 H, C*H*₂N(CH₃)₂), 3.09 und 3.25 (q und m, 10 H, CH=NC*H*₂CH₂C*H*₂NC*H*₂CH₂CH₂N(CH₃)₂ und N(C*H*₂CH₃)₂).
FT-IR: 2966, 2932, 2856, 2815, 2763, 1639, 1614, 1458, 1444, 1423, 1377, 1362, 1316, 1260, 1223, 1151, 1131, 1096, 2082, 1069, 1039, 974, 945, 870, 842, 792, 763.

### Amidin A2: 5-(1-(3-Dimethylaminopropyl)-1,4,5,6-tetrahydropyrimidin-2-yl)-N,N-dibutylpentanamid

In einem Rundkolben wurden 19.3 g (0.12 mol) 3-(3-(Dimethylamino)propylamino)propylamin (von BASF) in 30 ml Toluol vorgelegt und unter Rühren und Kühlen langsam 29.9 g (0.12 mol) N,N-Dibutyl-(2-oxocyclopentyl)carbonamid, hergestellt wie vorgängig beschrieben, zugetropft. Die Reaktionsmischung wurde über Nacht bei 40 °C gerührt. Anschliessend wurde das Azeotrop aus Toluol und Wasser bei 40 °C und 10 mbar abdestilliert. Dann wurde bei Normaldruck auf 145 bis 150 °C erhitzt und das restliche Toluol destillativ entfernt. Nach Abkühlen auf Raumtemperatur wurden 39.7 g Produkt als orange, geruchsfreie, niedrigviskose Flüssigkeit erhalten.
¹H-NMR (CDCl₃): 0.86 und 0.89 (2x t, 6 H, J= 7.1 Hz, N(CH₂CH₂CH₂C*H₃*)₂), 1.23 (m, 4 H, N(CH₂CH₂C*H*₂CH₃)₂), 1.43 (m, 4 H, N(CH₂C*H*₂CH₂CH₃)₂), 1.60 (m, 6 H, CH₂C*H*₂C*H*₂CH₂CO und CH₂C*H*₂CH₂N(CH₃)₂), 1.73 (m, 2 H, CH=NCH₂C*H*₂CH₂N), 2.13 (s, 6 H, N(CH₃)₂), 2.17 (m, 4 H, N=CHC*H*₂ und CH₂CO), 2.25 (m, 2 H, C*H*₂N(CH₃)₂), 3.10 und 3.24 (q und m, 10 H, CH=NC*H*₂CH₂C*H*₂NC*H*₂CH₂CH₂N(CH₃)₂ und N(C*H*₂CH₂CH₂CH₃)₂).
FT-IR: 2953, 2930, 2859, 2715, 2763, 1640, 1613, 1565, 1457, 1420, 1369, 1317, 1290, 1259, 1209, 1132, 1114, 1098, 1040, 974, 930, 869, 821, 785, 766, 732.

### Amidin A3: 5-(1-Methyl-1,4,5,6-tetrahydropyrimidin-2-yl)-N,N-dibutylpentanamid

In einem Rundkolben wurden 8.8 g (0.10 mol) N-Methyl-1,3-propandiamin in 50 ml Toluol vorgelegt und unter Rühren und Kühlen langsam 23.9 g (0.10 mol) N,N-Dibutyl-(2-oxocyclopentyl)carbonamid, hergestellt wie vorgängig beschrieben, zugetropft. Die Reaktionsmischung wurde 3 Stunden bei 40 °C gerührt. Anschliessend wurde das Azeotrop aus Toluol und Wasser bei 40 bis 50 °C und 1 mbar abdestilliert. Dann wurde bei Normaldruck langsam auf 150 °C erhitzt und das restliche Toluol destillativ entfernt. Nach Abkühlen auf Raumtemperatur wurden 27.6 g Produkt als orange, geruchsfreie, niedrigviskose Flüssigkeit erhalten.
¹H-NMR (CDCl₃): 0.86 (m, 6 H, N(CH₂CH₂CH₂C*H₃*)₂), 1.23 (m, 4 H, N(CH₂CH₂C*H*₂CH₃)₂), 1.43 (m, 4 H, N(CH₂C*H*₂CH₂CH₃)₂), 1.59 (m, 4 H, CH₂C*H₂*C*H₂*CH₂CO), 1.74 (m, 2 H, CH=NCH₂C*H₂*CH₂N), 2.20 und 2.26 (2x m, 4 H, N=CHC*H*₂ und C*H₂*CO), 2.81 (s, 3 H, N-C*H₃*), 3.06 und 3.13 (2x m, 4 H, NC*H₂*CH₂C*H*₂N), 3.23 (m, 4 H, N(C*H₂*CH₂CH₂CH₃)₂).
FT-IR: 2954, 2928, 2871, 2860, 1741, 1635, 1650, 1563, 1455, 1422, 1400, 1370, 1306, 1255, 1210, 1131, 1098, 1068, 1040, 954, 930, 903, 732.

### Amidin A4: 5-(1-Cocoalkyl-1,4,5,6-tetrahydropyrimidin-2-yl)-N,N-dibutylpentanamid

Amidin **A4** wurde hergestellt wie für Amidin **A3** beschrieben, wobei aber 25.7 g (0.10 mol) N-Cocoalkyl-1,3-propandiamin (Duomeen CD von Akzo Nobel) anstelle von N-Methyl-1,3-propandiamin eingesetzt wurden. Nach Abkühlen auf Raumtemperatur wurden 46.4 g Produkt als orange, geruchsfreie, niedrigviskose Flüssigkeit erhalten.
¹H-NMR (CDCl₃): 0.86 (m, 9 H, N(CH₂CH₂CH₂C*H₃*)₂und Alkylkette-CH₃), 1.22 (s *br,* 22 H, N(CH₂CH₂C*H₂*CH₃)₂ und (C*H*₂)₉), 1.47 (m, 6 H, N(CH₂C*H*₂CH₂CH₃)₂ und NCH₂*CH*₂-Alkylkette), 1.60 (m, 4 H, CH₂C*H₂*C*H₂*CH₂CO), 1.74 (m, 2 H, CH=NCH₂C*H₂*CH₂N), 2.18 und 2.28 (2x m, 4 H, N=CHC*H*₂ und C*H₂*CO), 3.10 (m, 6 H, NC*H₂*CH₂C*H₂*N und NC*H₂*-Alkylkette), 3.26 (m, 4 H, N(C*H₂*CH₂CH₂CH₃)₂).
FT-IR: 2955, 2922, 2852, 1643, 1615, 1566, 1464, 1455, 1419, 1370, 1317, 1288, 1265, 1207, 1130, 1100, 961, 930, 886, 765, 722.

**Amidin A5:** 5-(1,4,5,6-Tetrahydropyrimidin-2-yl)-N,N-dibutylpentanamid Amidin **A5** wurde hergestellt wie für Amidin **A3** beschrieben, wobei aber 7.4 g (0.12 mol) 1,3-Propandiamin anstelle von N-Methyl-1,3-propandiamin eingesetzt wurden. Nach Abkühlen auf Raumtemperatur wurden 25.7 g Produkt als orange, geruchsfreie, niedrigviskose Flüssigkeit erhalten.
¹H-NMR (CDCl₃): 0.86 (m, 6 H, N(CH₂CH₂CH₂C*H₃*)₂), 1.23 (m, 4 H, N(CH₂CH₂C*H₂*CH₃)₂), 1.43 (m, 4 H, N(CH₂C*H₂*CH₂CH₃)₂), 1.57 (m, 4 H, CH₂C*H₂*CH₂CH₂CO), 1.67 (m, 2 H, CH=NCH₂C*H₂*CH₂N), 2.05 und 2.25 (2x m, 4 H, N=CHC*H*₂ und C*H₂*CO), 3.12 (m, 4 H, NC*H₂*CH₂C*H*₂N), 3.23 (m, 4 H, N(C*H₂*CH₂CH₂CH₃)₂).
FT-IR: 3331, 3215, 2955, 2929, 2860, 1742, 1631, 1537, 1454, 1424, 1366, 1316, 1289, 1259, 1205, 1138, 1103, 1061, 980, 929, 902, 882, 837, 799, 732.

### Amidin A6: 5-(1-Methyl-1,4,5,6-tetrahydropyrimidin-2-yl)-pentancarbonsäuremethylester

Amidin **A6** wurde hergestellt wie für Amidin **A3** beschrieben, wobei aber 14.2 g (0.10 mol) 2-Oxocyclopentancarbonsäuremethylester anstelle von N,N-Dibutyl-(2-oxocyclopentyl)carbonamid eingesetzt wurden. Nach Abkühlen auf Raumtemperatur wurden 19.7 g Produkt als dunkle, geruchsfreie, niedrigviskose Flüssigkeit erhalten.
¹H-NMR (CDCl₃): 1.61 (m, 4 H, CH₂C*H₂*CH₂CH₂CO), 1.74 (m, 2 H, CH=NCH₂C*H₂*CH₂N), 2.18 und 2.26 (2x m, 4 H, N=CHC*H*₂ und C*H₂*CO), 2.80 (s, 3 H, N-*CH₃*), 3.06 und 3.23 (2x m, 4 H, NC*H₂*CH₂C*H*₂N), 3.59 (m, 3 H, O*CH₃*).
FT-IR: 3313, 3025, 2944, 2850, 2801, 1731, 1660, 1601, 1567, 1544, 1498, 1461, 1435, 1390, 1306, 1269, 1239, 1185, 1140, 1035, 1110, 1054, 1030, 917, 892, 844, 774, 728, 694.

### Amidin A7: Reaktionsgemisch enthaltend N,N'-((1,1,3,3-Tetramethyldisiloxan-1,3-diyl)bis(propan-3,1-diyl))bis(5-(1-methyl-1,4,5,6-tetrahydropyrimidin-2-yl)-pentanamid

In einem Rundkolben wurden 7.6 g (0.036 mol) Amidin **A6,** hergestellt wie vorgängig beschrieben, vorgelegt, unter Rühren 4.5 g (0.018 mol) Bis(3-aminopropyl)-1,1,3,3-tetramethyldisiloxan zugegeben und langsam unter Vakuum bis auf 160 °C erhitzt. Nach Abkühlen auf Raumtemperatur wurden 9.8 g einer dunklen, geruchsfreien, niedrigviskosen Flüssigkeit erhalten.
FT-IR: 3307, 2926, 2861, 1737, 1645, 1605, 1549, 1437, 1403, 1364, 1309, 1250, 1185, 1137, 1040, 837, 792, 774, 698.

### Amidin R1: 1-(3-Dimethylaminopropyl)-2-methyl-1,4,5,6-tetrahydropyrimidin

In einem Rundkolben wurden 131.6 g Ethylacetoacetat in 50 ml Toluol vorgelegt und unter Rühren und Kühlen langsam 161.1 g 3-(3-(Dimethylamino)propylamino)propylamin (von BASF) zugetropft, wobei die Temperatur bei 20 bis 30°C gehalten wurde. Die Reaktionsmischung wurde über Nacht bei 40°C gerührt. Darauf wurde das Azeotrop aus Toluol und Wasser bei 40°C und 10 mbar mittels Destillation aus der Reaktionsmischung entfernt und anschliessend das restliche Toluol und Ethylacetat mittels Destillation bei Normaldruck entfernt und der Rückstand im Vakuum destilliert. Man erhielt 168.7 g Produkt als gelbliche, geruchsfreie, niedrigviskose Flüssigkeit mit einer Siedetemperatur von 95 bis 105°C bei 0.6 mbar.

Die Amidine **A1** bis **A7** sind Amidine mit Struktureinheiten der Formel (I). Das Amidin **R1** ist ein Vergleichsbeispiel.

### Herstellung von Silangruppen aufweisenden Polyethern:

### Polymer STP-1:

Unter Feuchtigkeitsausschluss wurden 1000 g Polyol Acclaim® 12200 (Polyoxypropylendiol mit niedrigem Ungesättigtheitsgrad, von Covestro; OH-Zahl 11.0 mg KOH/g), 43.6 g Isophorondiisocyanat (IPDI; Vestanat® IPDI, von Evonik), 126.4 g Diisononyl-1,2-cyclohexandicarboxylat (DINCH) und 0.1 g Bismuttris(neodecanoat) (10 Gew.-% in DINCH) unter stetigem Rühren auf 90 °C aufgeheizt und auf dieser Temperatur belassen, bis der titrimetrisch bestimmte Gehalt an freien Isocyanatgruppen einen stabilen Wert von 0.63 Gew.-% erreicht hatte. Anschliessend wurden 63.0 g N-(3-Trimethoxysilylpropyl)aminobernsteinsäurediethylester (Addukt aus 3-Aminopropyltrimethoxysilan und Maleinsäurediethylester; hergestellt nach den Angaben in US 5,364,955) eingemischt und die Mischung bei 90 °C solange gerührt, bis mittels FT-IR-Spektroskopie kein freies Isocyanat mehr nachgewiesen wurde. Der so erhaltene Trimethoxysilangruppen aufweisende Polyether wurde auf Raumtemperatur abgekühlt und unter Ausschluss von Feuchtigkeit aufbewahrt.

### Polymer STP-2:

Unter Feuchtigkeitsausschluss wurden 1000 g Polyol Acclaim® 12200 (Polyoxypropylendiol mit niedrigem Ungesättigtheitsgrad, von Covestro; OH-Zahl 11.0 mg KOH/g), 43.6 g Isophorondiisocyanat (IPDI; Vestanat® IPDI, von Evonik), 126.4 g Diisononyl-1,2-cyclohexandicarboxylat (DINCH) und 0.1 g Bismuttris(neodecanoat) (10 Gew.-% in DINCH) unter stetigem Rühren auf 90 °C aufgeheizt und auf dieser Temperatur belassen, bis der titrimetrisch bestimmte Gehalt an freien Isocyanatgruppen einen stabilen Wert von 0.64 Gew.-% erreicht hatte. Anschliessend wurden 70.6 g N-(3-Triethoxysilylpropyl)aminobernsteinsäurediethylester (Addukt aus 3-Aminopropyltriethoxysilan und Maleinsäurediethylester) eingemischt und die Mischung bei 90°C solange gerührt, bis mittels FT-IR-Spektroskopie kein freies Isocyanat mehr nachgewiesen wurde. Der so erhaltene Triethoxysilangruppen aufweisende Polyether wurde auf Raumtemperatur abgekühlt und unter Ausschluss von Feuchtigkeit aufbewahrt.

### Verwendete kommerzielle Katalysatoren:

DBU 1,8-Diazabicyclo[5.4.0]undec-7-en (Lupragen® N 700, von BASF)
Zusammensetzungen auf Basis von Silangruppen aufweisenden Polymeren: Vergleichsbeispiele sind in den Tabellen 1 bis 4 mit "(Ref)" gekennzeichnet.

### Zusammensetzungen Z1 bis Z9:

Eine Zusammensetzung aus 97.6 g Polymer **STP-1,** 2.0 g Vinyltrimethoxysilan und 0.4 g 3-Aminopropyltrimethoxysilan wurde mit verschiedenen Katalysatoren in der angegebenen Menge gemäss Tabelle 1 vermengt und die Mischung auf Viskosität bei 25°C und Hautbildungszeit (HBZ) im Normklima, vor und nach Lagerung, geprüft. Die Hautbildungszeit dient dabei als Mass für die Aktivität des Katalysators in Bezug auf die Vernetzungsreaktion der Silangruppen, d.h. für die Vernetzungsgeschwindigkeit; die Veränderung der Viskosität und der Hautbildungszeit nach Lagerung sind ein Mass für die Lagerstabilität der Zusammensetzung. Weiterhin wurde die applizierte Mischung nach 24 h im Normklima daraufhin geprüft, ob die Oberfläche wie gewünscht trocken war oder sich ein schmieriger Film gebildet hatte, was ein Anzeichen für das Ausschwitzen des Katalysators aufgrund schlechter Verträglichkeit mit dem ausgehärteten Kunststoff ist, und/oder ob die Oberfläche klebrig war, was ein Anzeichen für eine unvollständige Aushärtung ist. Weiterhin wurde von der Mischung ein Film von 2 mm Dicke hergestellt, während 7 Tagen im Normklima ausgehärtet und auf mechanische Eigenschaften geprüft.
Die Ergebnisse sind in Tabelle 1 und 2 wiedergegeben.

**Tablle 1:**

| **Zusammensetzung** | **Katalysator** | **Menge** | **Konzentration¹** | **Viskosität [Pa·s]** | | **HBZ** | |
|---|---|---|---|---|---|---|---|
| | | | | **frisch** | **gelagert²** | **frisch** | **gelagert²** |
| **Z1** (Ref) | Amidin **R1** | 0.34 g | 1.9 | 21.3 | 22.2 | 31' | 32' |
| **Z2** (Ref) | DBU | 0.30 g | 1.9 | 26.3 | 31.0 | 29' | 31' |
| **Z3** | Amidin **A1** | 0.63 g | 1.9 | 24.9 | 41.2 | 25' | 25' |
| **Z4** | Amidin **A2** | 0.74 g | 1.9 | 24.5 | 41.9 | 25' | 25' |
| **Z5** | Amidin **A3** | 0.60 g | 1.9 | 46.5 | 49.3 | 36' | 27' |
| **Z6** | Amidin **A4** | 0.93 g | 1.9 | 44.5 | 47.0 | 35' | 30' |
| **Z7** | Amidin **A5** | 0.58 g | 1.9 | 46.2 | 48.7 | 25' | 21' |
| **Z8** | Amidin **A6** | 0.41 g | 1.9 | 34.1 | 37.7 | 38' | 40' |
| **Z9** | Amidin **A7** | 0.59 g | 1.9 | 27.9 | 31.1 | 33' | 40' |

| | | | | | | | |
|---|---|---|---|---|---|---|---|
| ¹ mmol Amidin-Gruppen auf 100 g Silangruppen aufweisender Polyether. ² während 7 Tagen bei 70 °C in geschlossenem Gebinde. | | | | | | | |

**Tabelle 2:**

| **Zusammensetzung** | **Oberfläche nach 24h** | **Zugfestigkeit** | **Bruchdehnung** | **E-Modul** | |
|---|---|---|---|---|---|
| | | | | **0-5%** | **0-50%** |
| **Z1** (Ref) | leicht klebrig | 0.62 MPa | 78% | 1.2 MPa | 0.8 MPa |
| **Z2** (Ref) | schmierig | 0.70 MPa | 95% | 1.0 MPa | 0.8 MPa |
| **Z3** | trocken | 0.69 MPa | 83% | 1.2 MPa | 0.8 MPa |
| **Z4** | trocken | 0.63 MPa | 72% | 1.2 MPa | 0.8 MPa |
| **Z5** | trocken | 0.71 MPa | 88% | 1.3 MPa | 0.8 MPa |
| **Z6** | trocken | 0.65 MPa | 84% | 1.2 MPa | 0.8 MPa |
| **Z7** | trocken | 0.65 MPa | 85% | 1.2 MPa | 0.8 MPa |
| **Z8** | trocken | 0.80 MPa | 108% | 1.2 MPa | 0.8 MPa |
| **Z9** | trocken | 0.79 MPa | 111% | 1.2 MPa | 0.8 MPa |

### Zusammensetzungen Z10 bis Z15:

In einem Planetenmischer wurden 36.2 g Polymer **STP-2,** 60.2 g gemahlene Kreide (Omyacarb® 5 GU, von Omya), 1.2 g Thixotropierpaste, deren Herstellung nachfolgend beschrieben ist, 1.2 g Vinyltriethoxysilan, 1.2 g 3-Aminopropyltriethoxysilan sowie verschiedene Katalysatoren in der angegebenen Menge gemäss Tabelle 3 vermengt und die Mischung wie für Zusammensetzung **Z1** beschrieben auf Viskosität, Hautbildungszeit (HBZ), Oberflächenbeschaffenheit und mechanische Eigenschaften geprüft. Die Ergebnisse sind in Tabelle 3 und 4 wiedergegeben.
Die Thixotropierpaste wurde hergestellt, indem in einem Vakuummischer 300 g Diisodecylphthalat (Palatinol® Z, von BASF) und 48 g 4,4'-Methylendiphenyldiisocyanat (Desmodur® 44 MC L, von Covestro) vorgelegt und leicht aufgewärmt und anschliessend unter starkem Rühren 27 g n-Butylamin langsam zugetropft wurden. Die entstehende Paste wurde unter Vakuum und Kühlung eine Stunde weitergerührt.

**Tabelle 3:**

| **Zusammensetzung** | **Katalysator** | **Menge** | **Konzentration¹** | **Viskosität [Pa·s]** | | **HBZ** | |
|---|---|---|---|---|---|---|---|
| | | | | **frisch** | **gelagert²** | **frisch** | **gelagert²** |
| **Z10** (Ref) | DBU | 0.40 g | 2.6 | n.b. | n.b. | 1h 23' | n.b. |
| **Z11** | Amidin **A1** | 0.84 g | 2.6 | 109 | 183 | 2h 45' | 5h |
| **Z12** | Amidin **A2** | 0.99 g | 2.6 | 105 | 179 | 3h | 2h |
| **Z13** | Amidin **A3** | 0.81 g | 2.6 | 139 | 167 | 4h | 5h |
| **Z14** | Amidin **A4** | 1.24 g | 2.6 | 130 | 154 | 3h 55' | 3h 30' |
| **Z15** | Amidin **A5** | 0.77 g | 2.6 | 157 | 175 | 3h 55' | 3h |

| | | | | | | | |
|---|---|---|---|---|---|---|---|
| ¹ mmol Amidin-Gruppen auf 100 g Zusammensetzung. ² während 7 Tagen bei 70 °C in geschlossenem Gebinde. "n. b." steht für "nicht bestimmt" | | | | | | | |

**Tabelle 4:**

| **Zusammensetzung** | **Oberflächenach 24h** | **Zugfestigkeit** | **Bruchdehnung** | **E-Modul** | |
|---|---|---|---|---|---|
| | | | | **0-5%** | **0-50%** |
| **Z10** (Ref) | schmierig | 2.5 MPa | 155% | 4.0 MPa | 2.0 MPa |
| **Z11** | trocken | 2.5 MPa | 141% | 4.4 MPa | 2.7 MPa |
| **Z12** | trocken | 2.3 MPa | 133% | 4.1 MPa | 2.5 MPa |
| **Z13** | trocken | 1.8 MPa | 143% | 3.2 MPa | 1.9 |
| **Z14** | trocken | 1.9 MPa | 137% | 3.6 MPa | 2.1 |
| **Z15** | trocken | 1.9 MPa | 155% | 3.7 MPa | 2.1 |

## Patentansprüche

1. Amidin enthaltend mindestens eine Struktureinheit der Formel (I), 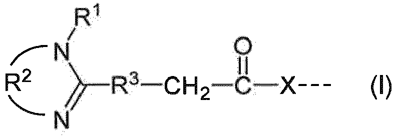 wobei
R¹ für einen Wasserstoff-Rest oder für einen gegebenenfalls ungesättigte Anteile aufweisenden Alkyl-, Cycloalkyl- oder Aralkyl-Rest mit 1 bis 30 C-Atomen, welcher gegebenenfalls eine tertiäre Aminogruppe aufweist, steht,
R² für einen gegebenenfalls Alkyl-substituierten 1,2-Ethylen- oder 1,3-Propylen-Rest steht,
R³ für einen gegebenenfalls Alkyl-substituierten 1,3-Propylen- oder 1,4-Butylen-Rest steht, und
X für O oder NR⁴ steht, wobei R⁴ für einen Wasserstoff-Rest oder für einen Alkyl-, Cycloalkyl- oder Aralkyl-Rest mit 1 bis 8 C-Atomen steht, **dadurch gekennzeichnet, dass** es die Formel (II) aufweist, 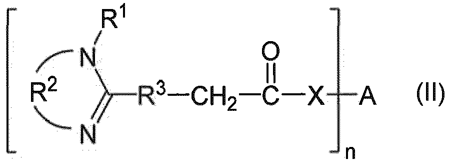 wobei
n für 1 oder 2 oder 3 steht, und
A für einen n-wertigen Rest mit einem Molekulargewicht im Bereich von 28 bis 5'000 g/mol, welcher über C-Atome gebunden ist, steht.

2. Amidin gemäss Anspruch 1, **dadurch gekennzeichnet, dass** R¹ ausgewählt ist aus der Gruppe bestehend aus Methyl, Ethyl, Propyl, Butyl, Cyclohexyl, 2-Ethylhexyl, n-Decyl, Lauryl, Cocoalkyl, Oleyl, Soyaalkyl, Talgalkyl und 3-(N,N-Dimethylamino)propyl.

3. Amidin gemäss einem der Ansprüche 1 oder 2, **dadurch gekennzeichnet, dass** X für NR⁴ steht.

4. Amidin gemäss einem der Ansprüche 1 bis 3, **dadurch gekennzeichnet, dass** A für einen einwertigen Kohlenwasserstoff-Rest mit 1 bis 30 C-Atomen, welcher gegebenenfalls Ether-Sauerstoff oder tertiären Amin-Stickstoff oder Siloxan-Einheiten enthält, steht.

5. Amidin gemäss einem der Ansprüche 1 bis 3, **dadurch gekennzeichnet, dass** A für einen zwei- oder dreiwertigen Kohlenwasserstoff-Rest mit 2 bis 50 C-Atomen, welcher gegebenenfalls Ether-Sauerstoff oder tertiären Amin-Stickstoff oder Siloxan-Einheiten enthält, steht.

6. Verfahren zur Herstellung des Amidins der Formel (II) gemäss einem der Ansprüche 1 bis 5, **dadurch gekennzeichnet, dass** mindestens ein Amin der Formel (III) mit einer Verbindung der Formel (IV) unter Entfernung von Wasser umgesetzt wird.

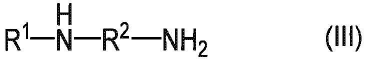

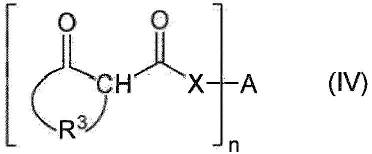

7. Verfahren gemäss Anspruch 6, **dadurch gekennzeichnet, dass** eine Verbindung der Formel (IV) mit X = NR⁴ eingesetzt wird, welche vorgängig durch Umsetzung von mindestens einem Amin der Formel A-(NHR⁴)ₙ mit einer Verbindung der Formel (IV) mit X = O unter Entfernung des entsprechenden Alkohols erhalten wurde.

8. Verwendung des Amidins gemäss einem der Ansprüche 1 bis 5 als Katalysator für die Vernetzung einer funktionellen Verbindung.

9. Verwendung gemäss Anspruch 8, **dadurch gekennzeichnet, dass** die funktionelle Verbindung ein Silan, ein Silangruppen aufweisendes Polymer, ein Polyisocyanat oder ein Isocyanatgruppen aufweisendes Polyurethanpolymer ist.

10. Härtbare Zusammensetzung umfassend mindestens ein Amidin gemäss einem der Ansprüche 1 bis 5.

11. Härtbare Zusammensetzung gemäss Anspruch 10, **dadurch gekennzeichnet, dass** sie Silangruppen und/oder Isocyanatgruppen enthält.

12. Härtbare Zusammensetzung gemäss einem der Ansprüche 10 oder 11, **dadurch gekennzeichnet, dass** sie mindestens ein Silangruppen aufweisendes Polymer enthält.

13. Härtbare Zusammensetzung gemäss einem der Ansprüche 10 bis 12, **dadurch gekennzeichnet, dass** sie einen Klebstoff oder einen Dichtstoff oder eine Beschichtung darstellt.

## Claims

1. Amidine containing at least one structural unit of the formula (I) 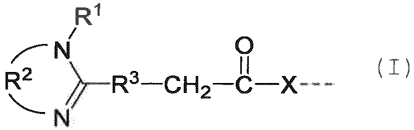 where
R¹ is a hydrogen radical or an alkyl, cycloalkyl or aralkyl radical optionally having unsaturated moieties, having 1 to 30 carbon atoms and optionally having a tertiary amino group,
R² is an optionally alkyl-substituted 1,2-ethylene or 1,3-propylene radical,
R³ is an optionally alkyl-substituted 1,3-propylene or 1,4-butylene radical, and
X is O or NR⁴ where R⁴ is a hydrogen radical or is an alkyl, cycloalkyl or aralkyl radical having 1 to 8 carbon atoms,
**characterized in that** it has the formula (II) 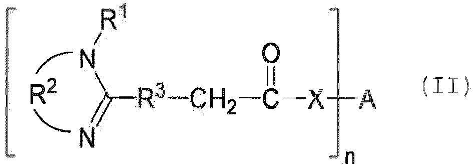 where
n is 1 or 2 or 3, and
A is an n-valent radical which has a molecular weight in the range from 28 to 5'000 g/mol and is bonded via carbon atoms.

2. Amidine according to Claim 1, **characterized in that** R¹ is selected from the group consisting of methyl, ethyl, propyl, butyl, cyclohexyl, 2-ethylhexyl, n-decyl, lauryl, cocoalkyl, oleyl, soyaalkyl, tallowalkyl and 3-(N,N-dimethylamino)propyl.

3. Amidine according to either of Claims 1 and 2, **characterized in that** X is NR⁴.

4. Amidine according to any of Claims 1 to 3, **characterized in that** A is a monovalent hydrocarbyl radical which has 1 to 30 carbon atoms and optionally contains ether oxygen or tertiary amine nitrogen or siloxane units.

5. Amidine according to any of Claims 1 to 3, **characterized in that** A is a di- or trivalent hydrocarbyl radical which has 2 to 50 carbon atoms and optionally contains ether oxygen or tertiary amine nitrogen or siloxane units.

6. Process for preparing the amidine of the formula (II) according to any of Claims 1 to 5, **characterized in that** at least one amine of the formula (III) is reacted with a compound of the formula (IV) with removal of water.

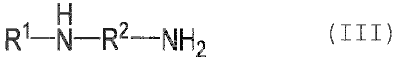

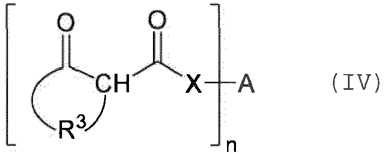

7. Process according to Claim 6, **characterized in that** a compound of the formula (IV) with X = NR⁴ is used, which has been obtained beforehand by reacting at least one amine of the formula A-(NHR⁴)ₙ with a compound of the formula (IV) with X = O with removal of the corresponding alcohol.

8. Use of the amidine according to any of Claims 1 to 5 as catalyst for the crosslinking of a functional compound.

9. Use according to Claim 8, **characterized in that** the functional compound is a silane, a polymer having silane groups, a polyisocyanate or a polyurethane polymer having isocyanate groups.

10. Curable composition comprising at least one amidine according to any of Claims 1 to 5.

11. Curable composition according to Claim 10, **characterized in that** it contains silane groups and/or isocyanate groups.

12. Curable composition according to either of Claims 10 and 11, **characterized in that** it comprises at least one polymer having silane groups.

13. Curable composition according to any of Claims 10 to 12, **characterized in that** it is an adhesive or a sealant or a coating.

## Revendications

1. Amidine contenant au moins une unité structurale de la formule (I) : 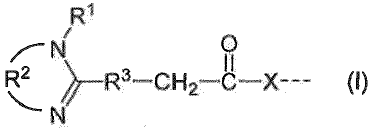 dans laquelle
R¹ représente un radical hydrogène ou un radical alkyle, cycloalkyle ou aralkyle de 1 à 30 atomes C, comprenant éventuellement des fractions insaturées, qui comprend éventuellement un groupe amino tertiaire,
R² représente un radical 1,2-éthylène ou 1,3-propylène éventuellement substitué par alkyle,
R³ représente un radical 1,3-propylène ou 1,4-butylène éventuellement susbtitué par alkyle, et
X représente O ou NR⁴, R⁴ représentant un radical hydrogène ou un radical alkyle, cycloalkyle ou aralkyle de 1 à 8 atomes C,
**caractérisée en ce qu'**elle présente la formule (II) 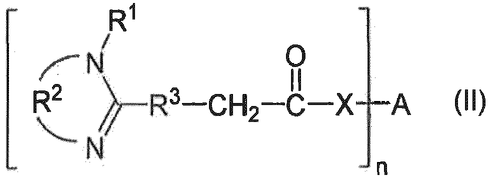 dans laquelle
n représente 1 ou 2 ou 3, et
A représente un radical n-valent ayant un poids moléculaire dans la plage allant de 28 à 5 000 g/mol, qui est relié par des atomes C.

2. Amidine selon la revendication 1, **caractérisée en ce que** R¹ est choisi dans le groupe constitué par méthyle, éthyle, propyle, butyle, cyclohexyle, 2-éthylhexyle, n-décyle, lauryle, cocoalkyle, oléyle, alkyle de soja, alkyle de suif et 3-(N,N-diméthylamino)propyle.

3. Amidine selon l'une quelconque des revendications 1 ou 2, **caractérisée en ce que** X représente NR⁴.

4. Amidine selon l'une quelconque des revendications 1 à 3, **caractérisée en ce qu'**A représente un radical hydrocarboné monovalent de 1 à 30 atomes C, qui contient éventuellement un oxygène d'éther ou un azote d'amine tertiaire ou des unités siloxane.

5. Amidine selon l'une quelconque des revendications 1 à 3, **caractérisée en ce qu'**A représente un radical hydrocarboné bi- ou trivalent de 2 à 50 atomes C, qui contient éventuellement un oxygène d'éther ou un azote d'amine tertiaire ou des unités siloxane.

6. Procédé de fabrication de l'amidine de la formule (II) selon l'une quelconque des revendications 1 à 5, **caractérisé en ce qu'**au moins une amine de la formule (III) est mise en réaction avec un composé de la formule (IV) avec élimination d'eau

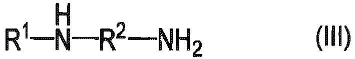

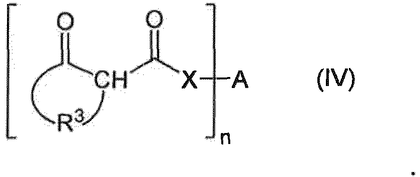

7. Procédé selon la revendication 6, **caractérisé en ce qu'**un composé de la formule (IV) avec X = NR⁴ est utilisé, qui a été obtenu auparavant par mise en réaction d'au moins une amine de la formule A-(NHR⁴)ₙ avec un composé de la formule (IV) avec X = 0 avec élimination de l'alcool correspondant.

8. Utilisation de l'amidine selon l'une quelconque des revendications 1 à 5 en tant que catalyseur pour la réticulation d'un composé fonctionnel.

9. Utilisation selon la revendication 8, **caractérisée en ce que** le composé fonctionnel est un silane, un polymère comprenant des groupes silane, un polyisocyanate ou un polymère de polyuréthane comprenant des groupes isocyanate.

10. Composition durcissable comprenant au moins une amidine selon l'une quelconque des revendications 1 à 5.

11. Composition durcissable selon la revendication 10, **caractérisée en ce qu'**elle contient des groupes silane et/ou des groupes isocyanate.

12. Composition durcissable selon l'une quelconque des revendications 10 ou 11, **caractérisée en ce qu'**elle contient au moins un polymère comprenant des groupes silane.

13. Composition durcissable selon l'une quelconque des revendications 10 à 12, **caractérisée en ce qu'**elle consiste en un adhésif ou un agent d'étanchéité ou un revêtement.
